# EUROPEAN PATENT SPECIFICATION

(11) **EP 1 763 538 B1**
(45) Date of publication and mention of the grant of the patent: **25.08.2010**
(21) Application number: 05763600.3
(22) Date of filing: 17.05.2005
(51) Int. Cl.: C07K 14/715, C07K 14/52, C12N 15/09, C12N 5/07

(54) **GENETICALLY ENGINEERED CELLS FOR THERAPEUTIC APPLICATIONS**
GENETISCH HERGESTELLTE ZELLEN FÜR THERAPEUTISCHE ANWENDUNGEN
CELLULES GENETIQUEMENT MODIFIEES POUR APPLICATIONS THERAPEUTIQUES

(30) Priority: 19.05.2004 US 572349 P
(43) Date of publication of application: 21.03.2007
(73) Proprietor: THE CLEVELAND CLINIC FOUNDATION, Cleveland, OH 44195 (US)
(72) Inventor: PENN, Marc, S., Shaker Heights, OH 44122 (US); KIEDROWSKI, Matthew, Cleveland, OH 44195 (US)
(74) Representative: Chapman, Paul Gilmour
(86) International application number: PCT/US2005/017050
(87) International publication number: WO 2005/116192

(56) References cited:
- WO-A-99/20759
- WO-A-2005/047494
- KAHN J ET AL: "Overexpression of CXCR4 on human CD34+ progenitors increases their proliferation, migration, and NOD/SCID repopulation" BLOOD, W.B.SAUNDERS COMPANY, ORLANDO, FL, US, vol. 103, no. 8, 15 April 2004 (2004-04-15), pages 2942-2949, XP002324814 ISSN: 0006-4971
- ASKARI A T ET AL: "Effect of stromal-cell-derived factor 1 on stem-cell homing and tissue regeneration in ischaemic cardiomyopathy" LANCET THE, LANCET LIMITED. LONDON, GB, vol. 362, no. 9385, 30 August 2003 (2003-08-30), pages 697-703, XP004778513 ISSN: 0140-6736
- PENN ET AL: "Autologous cell transplantation for the treatment of damaged myocardium" PROGRESS IN CARDIOVASCULAR DISEASES, SAUNDERS, PHILADELPHIA, PA, US, vol. 45, no. 1, July 2002 (2002-07), pages 21-32, XP005128836 ISSN: 0033-0620
- PENN M S ET AL: "Role of stem cell homing in myocardial regeneration" INTERNATIONAL JOURNAL OF CARDIOLOGY, ELSEVIER SCIENCE PUBLISHERS, AMSTERDAM, NL, vol. 95, June 2004 (2004-06), pages S23-S25, XP004546112 ISSN: 0167-5273

### SEQUENCE LISTING

### SEQUENCE LISTING

<110> Penn, Marc
<120> GENETICALLY ENGINEERED CELLS FOR THERAPEUTIC APPLICATIONS
<130> CCF-7019
<160> 9
<170> PatentIn version 3.2
<210> 1
   <211> 352
   <212> PRT
   <213> Homo sapiens
<400> 1
   Met Glu Gly Ile Ser Ile Tyr Thr Ser Asp Asn Tyr Thr Glu Glu Met
   1               5                   10                  15
   Gly Ser Gly Asp Tyr Asp Ser Met Lys Glu Pro Cys Phe Arg Glu Glu
               20                  25                  30
   Asn Ala Asn Phe Asn Lys Ile Phe Leu Pro Thr Ile Tyr Ser Ile Ile
           35                  40                  45
   Phe Leu Thr Gly Ile Val Gly Asn Gly Leu Val Ile Leu Val Met Gly
       50                  55                  60
   Tyr Gln Lys Lys Leu Arg Ser Met Thr Asp Lys Tyr Arg Leu His Leu
   65                  70                  75                  80
   Ser Val Ala Asp Leu Leu Phe Val Ile Thr Leu Pro Phe Trp Ala Val
                   85                  90                  95
   Asp Ala Val Ala Asn Trp Tyr Phe Gly Asn Phe Leu Cys Lys Ala Val
               100                 105                 110
   His Val Ile Tyr Thr Val Asn Leu Tyr Ser Ser Val Leu Ile Leu Ala
           115                 120                 125
   Phe Ile Ser Leu Asp Arg Tyr Leu Ala Ile Val His Ala Thr Asn Ser
       130                 135                 140
   Gln Arg Pro Arg Lys Leu Leu Ala Glu Lys Val Val Tyr Val Gly Val
   145                 150                 155                 160
   Trp Ile Pro Ala Leu Leu Leu Thr Ile Pro Asp Phe Ile Phe Ala Asn
                   165                 170                 175
   Val Ser Glu Ala Asp Asp Arg Tyr Ile Cys Asp Arg Phe Tyr Pro Asn
               180                 185                 190
   Asp Leu Trp Val Val Val Phe Gln Phe Gln His Ile Met Val Gly Leu
           195                 200                 205
   Ile Leu Pro Gly Ile Val Ile Leu Ser Cys Tyr Cys Ile Ile Ile Ser
       210                 215                 220
   Lys Leu Ser His Ser Lys Gly His Gln Lys Arg Lys Ala Leu Lys Thr
   225                 230                 235                 240
   Thr Val Ile Leu Ile Leu Ala Phe Phe Ala Cys Trp Leu Pro Tyr Tyr
                   245                 250                 255
   Ile Gly Ile Ser Ile Asp Ser Phe Ile Leu Leu Glu Ile Ile Lys Gln
               260                 265                 270
   Gly Cys Glu Phe Glu Asn Thr Val His Lys Trp Ile Ser Ile Thr Glu
           275                 280                 285
   Ala Leu Ala Phe Phe His Cys Cys Leu Asn Pro Ile Leu Tyr Ala Phe
       290                 295                 300
   Leu Gly Ala Lys Phe Lys Thr Ser Ala Gln His Ala Leu Thr Ser Val
   305                 310                 315                 320
   Ser Arg Gly Ser Ser Leu Lys Ile Leu Ser Lys Gly Lys Arg Gly Gly
                   325                 330                 335
   His Ser Ser Val Ser Thr Glu Ser Glu Ser Ser Ser Phe His Ser Ser
               340                 345                 350
<210> 2
   <211> 349
   <212> PRT
   <213> Rattus norvegicus
<400> 2
   Met Glu Ile Tyr Thr Ser Asp Asn Tyr Ser Glu Glu Val Gly Ser Gly
   1               5                   10                  15
   Asp Tyr Asp Ser Asn Lys Glu Pro Cys Phe Arg Asp Glu Asn Glu Asn
               20                  25                  30
   Phe Asn Arg Ile Phe Leu Pro Thr Ile Tyr Phe Ile Ile Phe Leu Thr
           35                  40                  45
   Gly Ile Val Gly Asn Gly Leu Val Ile Leu Val Met Gly Tyr Gln Lys
       50                  55                  60
   Lys Leu Arg Ser Met Thr Asp Lys Tyr Arg Leu His Leu Ser Val Ala
   65                  70                  75                  80
   Asp Leu Leu Phe Val Ile Thr Leu Pro Phe Trp Ala Val Asp Ala Met
                   85                  90                  95
   Ala Asp Trp Tyr Phe Gly Lys Phe Leu Cys Lys Ala Val His Ile Ile
               100                 105                 110
   Tyr Thr Val Asn Leu Tyr Ser Ser Val Leu Ile Leu Ala Phe Ile Ser
           115                 120                 125
   Leu Asp Arg Tyr Leu Ala Ile Val His Ala Thr Asn Ser Gln Ser Ala
       130                 135                 140
   Arg Lys Leu Leu Ala Glu Lys Ala Val Tyr Val Gly Val Trp Ile Pro
   145                 150                 155                 160
   Ala Leu Leu Leu Thr Ile Pro Asp Ile Ile Phe Ala Asp Val Ser Gln
                   165                 170                 175
   Gly Asp Gly Arg Tyr Ile Cys Asp Arg Leu Tyr Pro Asp Ser Leu Trp
               180                 185                 190
   Met Val Val Phe Gln Phe Gln His Ile Met Val Gly Leu Ile Leu Pro
           195                 200                 205
   Gly Ile Val Ile Leu Ser Cys Tyr Cys Ile Ile Ile Ser Lys Leu Ser
       210                 215                 220
   His Ser Lys Gly His Gln Lys Arg Lys Ala Leu Lys Thr Thr Val Ile
   225                 230                 235                 240
   Leu Ile Leu Ala Phe Phe Ala Cys Trp Leu Pro Tyr Tyr Val Gly Ile
                   245                 250                 255
   Ser Ile Asp Ser Phe Ile Leu Leu Glu Val Ile Lys Gln Gly Cys Glu
               260                 265                 270
   Phe Glu Ser Val Val His Lys Trp Ile Ser Ile Thr Glu Ala Leu Ala
           275                 280                 285
   Phe Phe His Cys Cys Leu Asn Pro Ile Leu Tyr Ala Phe Leu Gly Ala
       290                 295                 300
   Lys Phe Lys Ser Ser Ala Gln His Ala Leu Asn Ser Met Ser Arg Gly
   305                 310                 315                 320
   Ser Ser Leu Lys Ile Leu Ser Lys Gly Lys Arg Gly Gly His Ser Ser
                   325                 330                 335
   Val Ser Thr Glu Ser Glu Ser Ser Ser Phe His Ser Ser
               340                 345
<210> 3
   <211> 1662
   <212> RNA
   <213> Homo sapiens
<400> 3
   ccgagggcct gagtgctcca gtagccaccg catctggaga accagcggtt accatggagg     60
   ggatcagtat atacacttca gataactaca ccgaggaaat gggctcaggg gactatgact    120
   ccatgaagga accctgtttc cgtgaagaaa atgctaattt caataaaatc ttcctgccca    180
   ccatctactc catcatcttc ttaactggca ttgtgggcaa tggattggtc atcctggtca    240
   tgggttacca gaagaaactg agaagcatga cggacaagta caggctgcac ctgtcagtgg    300
   ccgacctcct ctttgtcatc acgcttccct tctgggcagt tgatgccgtg gcaaactggt    360
   actttgggaa cttcctatgc aaggcagtcc atgtcatcta cacagtcaac ctctacagca    420
   gtgtcctcat cctggccttc atcagtctgg accgctacct ggccatcgtc cacgccacca    480
   acagtcagag gccaaggaag ctgttggctg aaaaggtggt ctatgttggc gtctggatcc    540
   ctgccctcct gctgactatt cccgacttca tctttgccaa cgtcagtgag gcagatgaca    600
   gatatatctg tgaccgcttc taccccaatg acttgtgggt ggttgtgttc cagtttcagc    660
   acatcatggt tggccttatc ctgcctggta ttgtcatcct gtcctgctat tgcattatca    720
   tctccaagct gtcacactcc aagggccacc agaagcgcaa ggccctcaag accacagtca    780
   tcctcatcct ggctttcttc gcctgttggc tgccttacta cattgggatc agcatcgact    840
   ccttcatcct cctggaaatc atcaagcaag ggtgtgagtt tgagaacact gtgcacaagt    900
   ggatttccat caccgaggcc ctagctttct tccactgttg tctgaacccc atcctctatg    960
   ctttccttgg agccaaattt aaaacctctg cccagcacgc actcacctct gtgagcagag   1020
   ggtccagcct caagatcctc tccaaaggaa agcgaggtgg acattcatct gtttccactg   1080
   agtctgagtc ttcaagtttt cactccagct aacacagatg taaaagactt ttttttatac    1140
   gataaataac ttttttttaa gttacacatt tttcagatat aaaagactga ccaatattgt   1200
   acagttttta ttgcttgttg gatttttgtc ttgtgtttct ttagtttttg tgaagtttaa   1260
   ttgacttatt tatataaatt ttttttgttt catattgatg tgtgtctagg caggacctgt    1320
   ggccaagttc ttagttgctg tatgtctcgt ggtaggactg tagaaaaggg aactgaacat   1380
   tccagagcgt gtagtgaatc acgtaaagct agaaatgatc cccagctgtt tatgcataga   1440
   taatctctcc attcccgtgg aacgtttttc ctgttcttaa gacgtgattt tgctgtagaa   1500
   gatggcactt ataaccaaag cccaaagtgg tatagaaatg ctggtttttc agttttcagg   1560
   agtgggttga tttcagcacc tacagtgtac agtcttgtat taagttgtta ataaaagtac   1620
   atgttaaact taaaaaaaaa aaaaaaaaaa aaaaaaaaaa aa                       1662
<210> 4
   <211> 1050
   <212> RNA
   <213> Rattus norvegicus
<400> 4
   atggaaatat acacttcgga taactactcc gaagaagtag ggtctggaga ctatgactcc      60
   aacaaggaac cctgcttccg ggatgaaaac gaaaacttca acaggatctt cctgcccacc     120
   atctatttta tcatcttctt gactggcata gtgggcaatg ggttggtaat cctggtcatg     180
   ggttaccaga agaagctgag gagcatgaca gacaagtacc ggctgcacct gtccgtggct     240
   gacctcctct ttgtcatcac actccccttc tgggcagtgg acgccatggc tgactggtac     300
   tttgggaaat ttttatgtaa ggctgtgcat atcatctaca ccgtcaacct ttacagcagt     360
   gttctcatcc tggccttcat cagcctggac cgctaccttg ccattgtcca cgccaccaac     420
   agccagagcg cgaggaagct gctggctgaa aaggccgtct atgtgggtgt ctggatcccc     480
   gccctcctcc tgactatccc tgacatcatc ttcgccgatg tcagccaggg ggacggcagg     540
   tacatctgtg accgccttta ccccgacagc ctgtggatgg tggtgttcca gttccagcac     600
   atcatggtgg gtctcatcct gccgggcatc gtcatcctgt cctgttactg catcatcatc     660
   tccaagctgt cacactccaa gggccaccag aagcgcaagg ccctcaagac tacggtcatc     720
   cttatcctgg ctttctttgc ctgctggcta ccgtattacg tggggatcag catcgattcc     780
   ttcatccttt tggaggtcat caagcaagga tgtgagttcg agagcgtcgt gcacaagtgg     840
   atctccatca cggaggccct cgccttcttc cactgttgcc tgaaccccat cctctacgcc     900
   ttcctcgggg ccaaattcaa gagctccgcg cagcatgcac tcaattccat gagcagaggc     960
   tccagcctca agatcctttc caaagggaaa cggggtggac actcttccgt ctccacagag    1020
   tcagaatcct caagttttca ctccagctaa                                     1050
<210> 5
   <211> 68
   <212> PRT
   <213> Homo sapiens
<400> 5
   Lys Pro Val Ser Leu Leu Tyr Arg Cys Pro Cys Arg Phe Phe Glu Ser
   1               5                   10                  15
   His Val Ala Arg Ala Asn Val Lys His Leu Lys Ile Leu Asn Thr Pro
               20                  25                  30
   Asn Cys Ala Leu Gln Ile Val Ala Arg Leu Lys Asn Asn Asn Arg Gln
           35                  40                  45
   Val Cys Ile Asp Pro Lys Leu Lys Trp Ile Gln Glu Tyr Leu Glu Lys
       50                  55                  60
   Ala Leu Asn Lys
   65
<210> 6
   <211> 89
   <212> PRT
   <213> Homo sapiens
<400> 6
   Met Asn Ala Lys Val Val Val Val Leu Val Leu Val Leu Thr Ala Leu
   1               5                   10                  15
   Cys Leu Ser Asp Gly Lys Pro Val Ser Leu Ser Tyr Arg Cys Pro Cys
               20                  25                  30
   Arg Phe Phe Glu Ser His Val Ala Arg Ala Asn Val Lys His Leu Lys
           35                  40                  45
   Ile Leu Asn Thr Pro Asn Cys Ala Leu Gln Ile Val Ala Arg Leu Lys
       50                  55                  60
   Asn Asn Asn Arg Gln Val Cys Ile Asp Pro Lys Leu Lys Trp Ile Gln
   65                  70                  75                  80
   Glu Tyr Leu Glu Lys Ala Leu Asn Lys
                   85
<210> 7
   <211> 89
   <212> PRT
   <213> Rattus norvegicus
<400> 7
   Met Asp Ala Lys Val Val Ala Val Leu Ala Leu Val Leu Ala Ala Leu
   1               5                   10                  15
   Cys Ile Ser Asp Gly Lys Pro Val Ser Leu Ser Tyr Arg Cys Pro Cys
               20                  25                  30
   Arg Phe Phe Glu Ser His Val Ala Arg Ala Asn Val Lys His Leu Lys
           35                  40                  45
   Ile Leu Asn Thr Pro Asn Cys Ala Leu Gln Ile Val Ala Arg Leu Lys
       50                  55                  60
   Ser Asn Asn Arg Gln Val Cys Ile Asp Pro Lys Leu Lys Trp Ile Gln
   65                  70                  75                  80
   Glu Tyr Leu Asp Lys Ala Leu Asn Lys
                   85
<210> 8
   <211> 1940
   <212> RNA
   <213> Homo sapiens
<400> 8
   gccgcacttt cactctccgt cagccgcatt gcccgctcgg cgtccggccc ccgacccgcg     60
   ctcgtccgcc cgcccgcccg cccgcccgcg ccatgaacgc caaggtcgtg gtcgtgctgg    120
   tcctcgtgct gaccgcgctc tgcctcagcg acgggaagcc cgtcagcctg agctacagat    180
   gcccatgccg attcttcgaa agccatgttg ccagagccaa cgtcaagcat ctcaaaattc    240
   tcaacactcc aaactgtgcc cttcagattg tagcccggct gaagaacaac aacagacaag    300
   tgtgcattga cccgaagcta aagtggattc aggagtacct ggagaaagct ttaaacaagt    360
   aagcacaaca gccaaaaagg actttccgct agacccactc gaggaaaact aaaaccttgt    420
   gagagatgaa agggcaaaga cgtgggggag ggggccttaa ccatgaggac caggtgtgtg     480
   tgtggggtgg gcacattgat ctgggatcgg gcctgaggtt tgccagcatt tagaccctgc    540
   atttatagca tacggtatga tattgcagct tatattcatc catgccctgt acctgtgcac    600
   gttggaactt ttattactgg ggtttttcta agaaagaaat tgtattatca acagcatttt    660
   caagcagtta gttccttcat gatcatcaca atcatcatca ttctcattct cattttttaa    720
   atcaacgagt acttcaagat ctgaatttgg cttgtttgga gcatctcctc tgctcccctg    780
   gggagtctgg gcacagtcag gtggtggctt aacagggagc tggaaaaagt gtcctttctt    840
   cagacactga ggctcccgca gcagcgcccc tcccaagagg aaggcctctg tggcactcag    900
   ataccgactg gggctgggcg ccgccactgc cttcacctcc tctttcaacc tcagtgattg    960
   gctctgtggg ctccatgtag aagccactat tactgggact gtgctcagag acccctctcc   1020
   cagctattcc tactctctcc ccgactccga gagcatgctt aatcttgctt ctgcttctca   1080
   tttctgtagc ctgatcagcg ccgcaccagc cgggaagagg gtgattgctg gggctcgtgc   1140
   cctgcatccc tctcctccca gggcctgccc cacagctcgg gccctctgtg agatccgtct   1200
   ttggcctcct ccagaatgga gctggccctc tcctggggat gtgtaatggt ccccctgctt   1260
   acccgcaaaa gacaagtctt tacagaatca aatgcaattt taaatctgag agctcgcttt   1320
   gagtgactgg gttttgtgat tgcctctgaa gcctatgtat gccatggagg cactaacaaa   1380
   ctctgaggtt tccgaaatca gaagcgaaaa aatcagtgaa taaaccatca tcttgccact   1440
   accccctcct gaagccacag cagggtttca ggttccaatc agaactgttg gcaaggtgac 1500
   atttccatgc ataaatgcga tccacagaag gtcctggtgg tatttgtaac tttttgcaag 1560
   gcattttttt atatatattt ttgtgcacat ttttttttac gtttctttag aaaacaaatg 1620
   tatttcaaaa tatatttata gtcgaacaat tcatatattt gaagtggagc catatgaatg 1680
   tcagtagttt atacttctct attatctcaa actactggca atttgtaaag aaatatatat 1740
   gatatataaa tgtgattgca gcttttcaat gttagccaca gtgtattttt tcacttgtac 1800
   taaaattgta tcaaatgtga cattatatgc actagcaata aaatgctaat tgtttcatgg 1860
   tataaacgtc ctactgtatg tgggaattta tttacctgaa ataaaattca ttagttgtta 1920
   gtgatggagc ttaaaaaaaa 1940
<210> 9
   <211> 293
   <212> RNA
   <213> Rattus norvegicus
<400> 9
   ccatggacgc caaggtcgtc gctgtgctgg ccctggtgct ggccgcgctc tgcatcagtg 60
   acggtaagcc agtcagcctg agctacagat gcccctgccg attctttgag agccatgtcg 120
   ccagagccaa cgtcaaacat ctgaaaatcc tcaacactcc aaactgtgcc cttcagattg 180
   ttgcaaggct gaaaagcaac aacagacaag tgtgcattga cccgaaatta aagtggatcc 240
   aagagtacct ggacaaagcc ttaaacaagt aagcacaaca gcccaaagga ctt 293

## Description

### FIELD OF THE INVENTION

The present invention relates to genetically modified cells and, more particularly, to the use of genetically modified cells for therapeutic applications.

### BACKGROUND OF THE INVENTION

Acute myocardial infarction (MI) remains the leading cause of morbidity and mortality in western society. Despite recent therapeutic advances predominantly targeted at restoring antegrade perfusion in the infarct-related artery, a "ceiling" of benefit appears to exist. Topol, E.J. Lancet 357, 1905-1914 (2001). A substantial proportion of patients who experience an acute myocardial infarction (Ml) ultimately develop congestive heart failure (CHF) largely as a result of left ventricular (LV) remodeling, a process involving myocardial thinning, dilation, decreased function, ultimately leading to death. Robbins, M.A. & O'Connell, J.B., pp. 3-13 (Lippincott-Raven, Philadelphia, 1998). Pfeffer, J.M., Pfeffer, M.A., Fletcher, P.J. & Braunwald, E. Am. J. Physiol 260, H1406-H1414 (1991). Pfeffer, M.A. & Braunwald, E. Circulation 81, 1161-1172 (1990).

One method to treat this process following myocardial infarction involves cell therapy. Penn, M.S. et al. Prog. Cardiovasc. Dis. 45, 21-32 (2002). Cell therapy has focused on using a variety of cell types including differentiated cells, such as skeletal myoblasts, cardiac myocytes, smooth muscle cells, and fibroblasts, or bone marrow derived cells. Koh, G.Y., Klug, M.G., Soonpaa, M.H. & Field, L.J. J. Clin. Invest 92, 1548-1554 (1993). Taylor, D.A. et al. Nat. Med. 4, 929-933 (1998). Jain, M. et al. Circulation 103, 1920-1927 (2001). Li, R.K. et al. Ann. Thorac. Surg. 62, 654-660 (1996). Etzion, S. et al. J. Mol. Cell Cardiol. 33, 1321-1330 (2001). Li, R.K., Jia, Z.Q., Weisel, R.D., Merante, F. & Mickle, D.A. J. Mol. Cell Cardiol. 31, 513-522 (1999). Yoo, K.J. et al. Yonsei Med. J. 43, 296-303 (2002). Sakai, T. et al. Ann. Thorac. Surg. 68, 2074-2080 (1999). Sakai, T. et al. J. Thorac. Cardiovasc. Surg. 118, 715-724 (1999). Orlic, D. et al. Nature 410, 701-705 (2001). Tomita, S. et al. J. Thorac. Cardiovasc. Surg. 123, 1132-1140 (2002).

A growing body of literature suggests that stem cell mobilization to the heart and differentiation into cardiac myocytes is a naturally occurring process. Jackson, K.A. et al. J Clin. Invest 107, 1395-1402 (2001). Quaini, F. et al. N. Engl. J. Med. 346, 5-15 (2002). This process occurs at a rate insufficient to result in meaningful recovery of left ventricular function following myocardial infarction. *Id*. Recently, studies have demonstrated the possibility of regenerating damaged myocardium either through the direct injection of stem cells into the blood stream, or via chemical mobilization of stem cells from the bone marrow prior to the myocardial infarction. These studies have demonstrated the ability of stem cells to home to the infarct zone in the peri-infarct period, as well as for these cells to then differentiate into cardiac myocytes. Kocher, A.A. et al, Nat. Med. 7, 430-436 (2001). Orlic, D. et al. Proc. Natl. Acad. Sci. U. S. A 98, 10344-10349 (2001). Peled, A. et al. Blood 95, 3289-3296 (2000). Yong, K. et al. Br. J. Haematol. 107, 441-449 (1999). To date, all the studies have focused on the ability of stem cells to regenerate myocardium within 48 hours after myocardial infarction.

### SUMMARY OF THE INVENTION

The present invention relates to mesenchymal stem cells (MSCs), multipotent adult progenitor cells (MAPCs) and/or other stem cells capable of differentiating into specialized or partially specialized cell types of tissue or organ systems (*e.g*., cardiac structures including cardiac myocytes and blood vessels). The MSCs, MAPCs, and/or other stem cells in accordance with the invention are not human embryonic stem cells and are genetically engineered or modified to over-express a chemokine and/or chemokine receptor, which can substantially improve the survivability and longevity of the genetically modified MSCs, MAPCs, and/or other stem cells (*e.g*., used for the purpose of myocardial regeneration) as well as potentially improve the survivability of tissue in which the genetically modified stem cells are introduced. The over-expressed chemokine and/or chemokine receptor can mitigate apoptosis of the genetically modified stem cells when the genetically modified stem cells are introduced into a mammalian subject for therapeutic applications and/or cellular therapy (*e.g*., regenerating myocardial tissue, recovering myocardial function, or preserving cardiac function in congestive heart failure and/or acute myocardial infarction). The over-expressed chemokine and/or chemokine receptor can also potentially mitigate apoptosis in tissue of the mammalian subject treated with the genetically modified MSCs, MAPCs, and/or other stem cells (*e.g.,* used for myocardial regeneration).

In one aspect of the invention, the stem cells can be genetically modified to over-express CXC chemokine receptor 4 (CXCR4). The stem cells that over-express CXCR4 can be directly injected into myocardial tissue or venous or arterial infused to treat a recent myocardial infarction or congestive heart failure. The over-expression of CXCR4 from the MSCs, MAPCs, and/or other stem cells can potentially improve the survivability of the MSCs, MAPCs and/or other stem cells in the myocardial tissue and the homing of the MSCs, MAPCs, and/or other stem cells, such as hematopoietic stem cells, to SDF-1 and/or cells expressing SDF-1.

In accordance with another aspect of the invention, the MSCs, MAPCs, and/or other stem cells (*e.g*., used for myocardial regeneration) can be genetically modified to over-express SDF-1. The stem cells that over-express SDF-1 can be directly injected into myocardial tissue or venous or arterial infused to treat a recent myocardial infarction or congestive heart failure. SDF-1 that is expressed from the stem cells can potentially induce stem cells in the peripheral blood to home to infarcted myocardium. Moreover, the over-expression of SDF-1 can substantially improve the survival of the MSCs, MAPCs, and/or other stem cells (*e.g*., used for myocardial regeneration) as well as other cells proximate the MSCs and/or MAPCs.

In yet another aspect of the invention, the MSCs, MAPCs, and/or other stem cells can be genetically modified to over-express both CXCR4 and SDF-1. The stem cells that over-express both CXCR4 and SDF-1 can be directly injected into myocardial tissue or venous or arterial infused to treat a recent myocardial infarction or congestive heart failure. CXCR4 and SDF-1 that is expressed from the stem cells can potentially induce stem cells in the peripheral blood to home to infarcted myocardium. Moreover, the over-expression of CXCR4 and SDF-1 can substantially improve the survival of the MSCs, MAPCs, and/or other stem cells used for myocardial regeneration as well as other cells proximate to the stem cells.

### BRIEF DESCRIPTION OF THE DRAWINGS

The foregoing and other aspects of the present invention will become apparent to those skilled in the art to which the present invention relates upon reading the following description with reference to the accompanying drawings.
FIG. 1 is schematic block diagram illustrating a clinical strategy using MSCs and/or MAPCs and/or other stem cells genetically modified to over-express CXCR4 and SDF-1 in accordance with the present invention.
FIG. 2 is a graph illustrating the number of MSCs and MSCs genetically modified to express CXCR4 in the infarct zone per unit area three days after infusion of the MSCs and genetically modified MSCs in a rat.
FIG. 3 is western blot analysis illustrating that the AKT of rat MSCs stably transfected to express CXCR4 or SDF-1 was readily phosphorylated in comparison to the AKT of MSCs not transfected.
FIG. 4A are photographs showing representative sections of the infarct zone of respective rats three days following infusion of the control MSCs and the MSCs expressing SDF-1 or CXCR4.
FIG. 4B is graph that compares the number of control MSCs with the number of MSCs genetically modified to express CXCR4 or SDF-1 in the infarct zones per unit area three days after infusion.
FIG. 5 are photographs of the respective infarct zones of rats treated with control MSCs and MSCs expressing SDF-1 four days following myocardial infarction.
FIG. 6 is a graph showing the improvement in LV function fourteen days following myocardial infarction for rats implanted with saline, cardiac fibroblast, control MSCs, MSCs expressing SDF-1, and MSCs expressing CXCR4.

### DETAILED DESCRIPTION

Unless otherwise defined, all technical terms used herein have the same meaning as commonly understood by one of ordinary skill in the art to which this invention belongs. Commonly understood definitions of molecular biology terms can be found in, for example, Rieger et al., Glossary of Genetics: Classical and Molecular, 5th edition, Springer-Verlag: New York, 1991; and Lewin, Genes V, Oxford University Press: New York, 1994.

Methods involving conventional molecular biology techniques are described herein. Such techniques are generally known in the art and are described in detail in methodology treatises, such as Molecular Cloning: A Laboratory Manual, 2nd ed., vol. 1-3, ed. Sambrook et al., Cold Spring Harbor Laboratory Press, Cold Spring Harbor, N.Y., 1989; and Current Protocols in Molecular Biology, ed. Ausubel et al., Greene Publishing and Wiley-Interscience, New York, 1992 (with periodic updates). Methods for chemical synthesis of nucleic acids are discussed, for example, in Beaucage and Carruthers, Tetra. Letts. 22:1859-1862, 1981, and Matteucci et al., J. Am. Chem. Soc. 103:3185, 1981. Chemical synthesis of nucleic acids can be performed, for example, on commercial automated oligonucleotide synthesizers. Immunological methods (*e.g*., preparation of antigen-specific antibodies, immunoprecipitation, and immunoblotting) are described, *e.g*., in Current Protocols in Immunology, ed. Coligan et al., John Wiley & Sons, New York, 1991; and Methods of Immunological Analysis, ed. Masseyeff et al., John Wiley & Sons, New York, 1992. Conventional methods of gene transfer and gene therapy can also be adapted for use in the present invention. See, *e.g*., Gene Therapy: Principles and Applications, ed. T. Blackenstein, Springer Verlag, 1999; Gene Therapy Protocols (Methods in Molecular Medicine), ed. P. D. Robbins, Humana Press, 1997; and Retro-vectors for Human Gene Therapy, ed. C. P. Hodgson, Springer Verlag, 1996.

The present invention relates to mesenchymal stem cells (MSCs), multipotent adult progenitor cells (MAPCs), and/or other stem cells capable of differentiating into specialized or partially specialized cell types of tissue or organ systems (*e.g*., used to regenerate myocardial structures including cardiac myocytes and blood vessels). The MSCs, MAPCs, and/or other stem cells according to this invention are not human embryonic stem cells and are genetically engineered or modified to over-express a chemokine and/or chemokine receptor, which can substantially improve the survivability and longevity of the genetically modified these stem cells as well as potentially improve the survivability of tissue in which the genetically modified stem cells are introduced. The over-expressed chemokine and/or chemokine receptor can mitigate apoptosis of the genetically modified MSCs, MAPCs and/or other stem cells (*e.g*., used to regenerate myocardial structures) when the genetically modified stem cells are introduced into a mammalian subject for therapeutic applications and/or cellular therapy (*e.g*., regenerating myocardial tissue, recovering myocardial function, or preserving cardiac function in congestion heart failure and/or acute myocardial infarction). The over-expressed chemokine and/or chemokine receptor can also potentially mitigate apoptosis in tissue of the mammalian subject treated with the genetically modified MSCs, MAPCs, and/or other stem cells.

Mammalian subjects can include any mammal, such as human beings, rats, mice, cats, dogs, goats, sheep, horses, monkeys, apes, rabbits, cattle, etc. The mammalian subject can be in any stage of development including adults, young animals, and neonates. Mammalian subjects can also include those in a fetal stage of development.

The MSCs in accordance with the present invention are not human embryonic stem cells and are the formative pluripotent blast or embryonic cells that differentiate into the specific types of connective tissues, (*i.e.,* the tissue of the body that support specialized elements, particularly including adipose, osseous, cartilaginous, elastic, muscular, and fibrous connective tissues depending on various *in vivo* or *in vitro* environmental influences). These cells are present in bone marrow, blood, dermis, and periosteum and can be isolated and purified using various well known methods, such as those methods disclosed in U.S. Patent No. 5,197,985 to Caplan and Haynesworth, as well as other numerous literature references.

The MAPCs in accordance with the present invention are not human embryonic stem cells and comprise adult progenitor or stem cells that are capable of differentiating into cells types beyond those of the tissues in which they normally reside (*i.e*., exhibit plasticity). Examples of MAPCs can include adult MSCs and hematopoietic progenitor cells. Sources of MAPCs can include bone marrow, blood, ocular tissue, dermis, liver, and skeletal muscle. By way of example, MAPCs comprising hematopoietic progenitor cells can be isolated and purified using the methods disclosed in U.S. Patent No. 5,061,620 as well as other numerous literature sources.

Other stem cell types in accordance with the present invention comprise adult progenitor or stem cells that are capable of differentiating into specialized or partially specializes cells for tissues or organ systems. In an aspect of the invention, the other stem cell types can comprises adult progenitor or stem cells that are capable of differentiating into cardiac myocytes or other myocardial structures including blood vessels, and in which the over-expression of CXCR4 and/or SDF-1 results in (i) increased survival of the stem cells, (ii) increased homing and/or engraftment of the modified stem cells into the infarcted myocardium, (iii) increased survival or decreased apoptosis in the tissue surrounding engrafted stem cells, and/or (iv) increased homing of endogenous circulating stem cells into the infarcted myocardium.

### CXCR4 over-expression

In one aspect of the invention, the MSCs, MAPCs, and/or other stem cells can be genetically modified to over-express CXC chemokine receptor 4 (CXCR4). CXCR4, also known as CD184, leukocyte-derived seven transmembrane domain receptor(LESTR), neuropeptide Y receptor Y3 (NPY3R), HM89, and FB22, is a G protein-coupled receptor with selectivity for a single CXC-motif chemokine, called stromal cell-derived factor-1 (SDF1). CXCR4 mediates chemotaxis in mature and progenitor blood cells and, together with its ligand SDF-1, is essential for B lympho- and myelopoiesis. In addition to hematopoiesis, CXCR4 is responsible for cardiac ventricular septum formation, vascularization of the gastrointestinal tract and development of cerebellar granule cells.

The amino acid sequences of a number of different mammalian CXCR4 polypeptides (or proteins) are known including human, mouse, and rat. These polypeptides typically include 352 amino acids. The CXCR4 polypeptide that is over-expressed in accordance with the present can have an amino acid sequence substantially similar to one of the foregoing mammalian CXCR4 polypeptides. For example, the CXCR4 that is over-expressed can have an amino acid sequence substantially similar to SEQ ID NO: 1. SEQ ID NO: 1 comprises the amino sequence for human CXCR4 and is identified by GenBank Accession No. P61073. The CXCR4 that is over-expressed can also have an amino acid sequence that is substantially similar to SEQ ID NO: 2. SEQ ID NO: 2 comprises the amino acid sequences for mouse CXCR4 and is identified by GenBank Accession No. 008565.

The CXCR4 that is over-expressed in accordance with the present invention can also be a variant of mammalian CXCR4, such as a fragment, analog and derivative of mammalian CXCR4. Such variants include, for example, a polypeptide encoded by a naturally occurring allelic variant of native CXCR4 gene (*i.e.,* a naturally occurring nucleic acid that encodes a naturally occurring mammalian CXCR4 polypeptide), a polypeptide encoded by an alternative splice form of a native CXCR4 gene, a polypeptide encoded by a homolog or ortholog of a native CXCR4 gene, and a polypeptide encoded by a non-naturally occurring variant of a native CXCR4 gene.

CXCR4 variants have a peptide sequence that differs from a native CXCR4 polypeptide in one or more amino acids. The peptide sequence of such variants can feature a deletion, addition, or substitution of one or more amino acids of a CXCR4 variant. Amino acid insertions are preferably of about 1 to 4 contiguous amino acids, and deletions are preferably of about 1 to 10 contiguous amino acids. Variant CXCR4 polypeptides substantially maintain a native CXCR4 functional activity. Preferred CXCR4 polypeptide variants can be made by expressing nucleic acid molecules within the invention that feature silent or conservative changes.

CXCR4 polypeptide fragments corresponding to one or more particular motifs and/or domains or to arbitrary sizes, are within the scope of the present invention. Isolated peptidyl portions of CXCR4 can be obtained by screening peptides recombinantly produced from the corresponding fragment of the nucleic acid encoding such peptides. For example, a CXCR4 polypetides of the present invention may be arbitrarily divided into fragments of desired length with no overlap of the fragments, or preferably divided into overlapping fragments of a desired length. The fragments can be produced recombinantly and tested to identify those peptidyl fragments, which can function as agonists of native CXCR4 polypeptides.

Variants of CXCR4 polypeptides can also include recombinant forms of the CXCR4 polypeptides. Recombinant polypeptides preferred by the present invention, in addition to a CXCR4 polypeptides, are encoded by a nucleic acid that can have at least about 70% sequence identity with the nucleic acid sequence of a gene encoding a mammalian CXCR4 polypeptides.

CXCR4 polypeptides variants can include agonistic forms of the protein that constitutively express the functional activities of a native CXCR4 polypeptide. Other CXCR4 polypeptide variants can include those that are resistant to proteolytic cleavage, as for example, due to mutations, which alter protease target sequences. Whether a change in the amino acid sequence of a peptide results in a variant having one or more functional activities of a native CXCR4 polypeptides can be readily determined by testing the variant for a native CXCR4 polypeptide functional activity.

The MSCs, MAPCs, and/or other stem cells in accordance with the present invention can be genetically modified with a nucleic acid that encodes CXCR4 or a variant of CXCR4. The nucleic acid can be a native or non-native nucleic acid and be in the form ofRNA or in the form of DNA (*e.g*., cDNA, genomic DNA, and synthetic DNA). The DNA can be double-stranded or single-stranded, and if single-stranded may be the coding (sense) strand or non-coding (anti-sense) strand. The nucleic acid coding sequence that encodes a CXCR4 polypeptide may be substantially similar to the nucleotide sequence of a CXCR4 gene, such as a nucleotide sequence shown in SEQ ID NO: 3 and SEQ ID NO: 4. SEQ ID NO: 3 and SEQ ID NO: 4 comprise, respectively, the nucleic acid sequences for human CXCR4 and rat CXCR4 and are substantially similar to the nucleic sequences of GenBank Accession No. BC020968 and GenBank Accession No. BC031655. The nucleic acid coding sequence of CXCR4 can also be a different coding sequence which, as a result of the redundancy or degeneracy of the genetic code, encodes the same polypeptide as SEQ ID NO: 3 and SEQ ID NO: 4.

Other nucleic acid molecules that encode CXCR4 within the invention are variants of a native CXCR4 gene, such as those that encode fragments, analogs and derivatives of a native CXCR4 polypeptide. Such variants may be, for example, a naturally occurring allelic variant of a native CXCR4 gene, a homolog or ortholog of a native CXCR4 gene, or a non-naturally occurring variant of a native CXCR4 gene. These variants have a nucleotide sequence that differs from a native CXCR4 gene in one or more bases. For example, the nucleotide sequence of such variants can feature a deletion, addition, or substitution of one or more nucleotides of a native CXCR4 gene. Nucleic acid insertions are preferably of about 1 to 10 contiguous nucleotides, and deletions are preferably of about 1 to 10 contiguous nucleotides.

In other applications, variant CXCR4 polypeptides displaying substantial changes in structure can be generated by making nucleotide substitutions that cause less than conservative changes in the encoded polypeptide. Examples of such nucleotide substitutions are those that cause changes in (a) the structure of the polypeptide backbone; (b) the charge or hydrophobicity of the polypeptide; or (c) the bulk of an amino acid side chain. Nucleotide substitutions generally expected to produce the greatest changes in protein properties are those that cause non-conservative changes in codons. Examples of codon changes that are likely to cause major changes in polypeptide structure are those that cause substitution of (a) a hydrophilic residue (*e.g*., serine or threonine) for (or by) a hydrophobic residue (*e.g*., leucine, isoleucine, phenylalanine, valine or alanine) (b) a cysteine or proline for (or by) any other residue; (c) a residue having an electropositive side chain (*e.g*., lysine, arginine, or histidine) for (or by) an electronegative residue (*e.g*., glutamine or aspartine); or (d) a residue having a bulky side chain (*e.g*., phenylalanine) for (or by) one not having a side chain (*e.g*., glycine).

Naturally occurring allelic variants of a native CXCR4 gene within the invention are nucleic acids isolated from mammalian tissue that have at least about 70% sequence identity with a native CXCR4 gene, and encode polypeptides having structural similarity to a native CXCR4 polypeptide. Homologs of a native CXCR4 gene within the invention are nucleic acids isolated from other species that have at least about 70% sequence identity with the native gene, and encode polypeptides having structural similarity to a native CXCR4 polypeptide. Public and/or proprietary nucleic acid databases can be searched to identify other nucleic acid molecules having a high percent (*e.g*., 70% or more) sequence identity to a native CXCR4 gene.

Non-naturally occurring CXCR4 gene variants are nucleic acids that do not occur in nature (*e.g*., are made by the hand of man), have at least about 70% sequence identity with a native CXCR4 gene, and encode polypeptides having structural similarity to a native CXCR4 polypeptide. Examples of non-naturally occurring CXCR4 gene variants are those that encode a fragment of a native CXCR4 protein, those that hybridize to a native CXCR4 gene or a complement of to a native CXCR4 gene under stringent conditions, and those that share at least 65% sequence identity with a native CXCR4 gene or a complement of a native CXCR4 gene.

Nucleic acids encoding fragments of a native CXCR4 gene within the invention are those that encode, amino acid residues of a native CXCR4 polypeptide. Shorter oligonucleotides that encode or hybridize with nucleic acids that encode fragments of a native CXCR4 polypeptide can be used as probes, primers, or antisense molecules. Longer polynucleotides that encode or hybridize with nucleic acids that encode fragments of a native CXCR4 polypeptide can also be used in various aspects of the invention. Nucleic acids encoding fragments of a native CXCR4 can be made by enzymatic digestion (*e.g*., using a restriction enzyme) or chemical degradation of the full length native CXCR4 gene or variants thereof.

Nucleic acids that hybridize under stringent conditions to one of the foregoing nucleic acids can also be used in the invention. For example, such nucleic acids can be those that hybridize to one of the foregoing nucleic acids under low stringency conditions, moderate stringency conditions, or high stringency conditions are within the invention.

Nucleic acid molecules encoding a CXCR4 fusion protein may also be used in the invention. Such nucleic acids can be made by preparing a construct (*e.g*., an expression vector) that expresses a CXCR4 fusion protein when introduced into a suitable target cell. For example, such a construct can be made by ligating a first polynucleotide encoding a CXCR4 protein fused in frame with a second polynucleotide encoding another protein such that expression of the construct in a suitable expression system yields a fusion protein.

The nucleic acids used to over-express CXCR4 can be modified at the base moiety, sugar moiety, or phosphate backbone, for example, to improve stability of the molecule, hybridization, etc. The nucleic acids within the invention may additionally include other appended groups such as peptides (*e.g*., for targeting target cell receptors *in vivo*)*,* or agents facilitating transport across the cell membrane, hybridization-triggered cleavage. To this end, the nucleic acids may be conjugated to another molecule (*e.g*., a peptide, hybridization triggered crosslinking agent, transport agent, hybridization-triggered cleavage agent, etc).

The CXCR4 can be over-expressed from the MSCs, MAPCs, and/or other stem cells by introducing an agent into the stem cells, during, for example, culturing of the MSCs, MAPCs, and/or other stem cells that promote expression of CXCR4. The agent can comprise natural or synthetic nucleic acids (*e.g*., exogenous genetic material), according to present invention and described above, that are incorporated into recombinant nucleic acid constructs, typically DNA constructs, capable of introduction into and replication in the cell. Such a construct preferably includes a replication system and sequences that are capable of transcription and translation of a polypeptide-encoding sequence in a given target cell.

Other agents can also be introduced into the MSCs, MAPSs, and/or other stem cells to promote expression of CXCR4 from the stem cells. For example, agents that increase the transcription of a gene encoding CXCR4, increase the translation of an mRNA encoding CXCR4, and/ or those that decrease the degradation of an mRNA encoding CXCR4 could be used to increase CXCR4 levels. Increasing the rate of transcription from a gene within a cell can be accomplished by introducing an exogenous promoter upstream of the gene encoding CXCR4. Enhancer elements, which facilitate expression of a heterologous gene, may also be employed.

A preferred method of introducing the agent into a MSCs, MAPCs, and/or other stem cells involves using gene therapy. Gene therapy refers to gene transfer to express a therapeutic product from a cell *in vivo, ex vivo,* or *in vitro.* Gene therapy in accordance with the present invention can be used to express CXCR4 from the MSCs, MAPCs, and/or other stem cells *in vivo, ex vivo,* and/or *in vitro.*

One method of gene therapy uses a vector including a nucleotide encoding a CXCR4. A "vector" (sometimes referred to as gene delivery or gene transfer "vehicle") refers to a macromolecule or complex of molecules comprising a polynucleotide to be delivered to a target cell, either *in vitro* or *in vivo.* The polynucleotide to be delivered may comprise a coding sequence of interest in gene therapy. Vectors include, for example, viral vectors (such as adenoviruses ('Ad'), adeno-associated viruses (AAV), lentiviruses and retroviruses), liposomes and other lipid-containing complexes, and other macromolecular complexes capable of mediating delivery of a polynucleotide to a target cell *(i.e.,* MSCs, MAPCs, and/or other stem cells).

Vectors can also comprise other components or functionalities that further modulate gene delivery and/or gene expression, or that otherwise provide beneficial properties to the targeted cells. Such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors, which have components or functionalities mediating binding and uptake), or vectors can be modified to provide such functionalities.

Selectable markers can be positive, negative or bifunctional. Positive selectable markers allow selection for cells carrying the marker, whereas negative selectable markers allow cells carrying the marker to be selectively eliminated. A variety of such marker genes have been described, including bifunctional (*i.e*. positive/negative) markers (see, *e.g*., Lupton, S., WO 92/08796, published May 29, 1992; and Lupton, S., WO 94/28143, published Dec. 8, 1994). Such marker genes can provide an added measure of control that can be advantageous in gene therapy contexts. A large variety of such vectors are known in the art and are generally available.

Vectors that can be used in the present invention include viral vectors, lipid based vectors and other vectors that are capable of delivering a nucleotide according to the present invention to the MSCs, MAPCs, and/or other stem cells. The vector can be a targeted vector, especially a targeted vector that preferentially binds to MSCs, MAPCs and/or other stem cells. Preferred viral vectors for use in the invention are those that exhibit low toxicity to a target cell and induce production of therapeutically useful quantities of CXCR4.

One example of a viral vector that can be used to genetically modify MSCs, MAPCs, and/or other stem cells is a retrovirus. The use of retroviruses for genetically modifying MSCs is disclosed in U.S. Patent No. 5,591,625. The structure and life cycle of retroviruses makes them ideally suited to be gene-transfer vehicles since (i) the majority of sequences coding for their structural genes are deleted and replaced by the gene(s) of interest, which are transcribed under control of the retroviral regulatory sequences within its long terminal repeat (LTR) region and (ii) they replicate through a DNA intermediate that integrates into the host genome. Although the sites of integration appear to be random with respect to the host genome, the provirus integrates with a defined structure in low copy number.

Retroviruses can be RNA viruses; *i.e*., the viral genome is RNA. This genomic RNA is, however, reverse transcribed into a DNA intermediate which is integrated very efficiently into the chromosomal DNA of infected cells. This integrated DNA intermediate is referred to as a provirus. The retroviral genome and the proviral DNA have three genes: the gag, the pol and the env, which are flanked by two long terminal repeat (LTR) sequences. The gag gene encodes the internal structural (nucleocapsid) proteins, the pol gene encodes the RNA-directed DNA polymerase (reverse transcriptase); and the env gene encodes viral envelope glycoproteins. The 5' and 3' LTRs serve to promote transcription and polyadenylation of virion RNAs.

Adjacent to the 5' LTR are sequences necessary for reverse transcription of the genome (the tRNA primer binding site) and for efficient encapsidation of viral RNA into particles (the Psi site). Mulligan, R. C., In: Experimental Manipulation of Gene Expression, M. Inouye (ed). Proceedings of the National Academy of Sciences, U.S.A. 81:6349-6353 (1984).

In order to generate a viral particle containing the recombinant genome, it is necessary to develop cell lines that provide packaging "help". To accomplish this, a plasmid(s), encoding, for example, the retroviral structural genes gag, pol, and env, is introduced into an otherwise untransformed tissue cell line by conventional calcium-phosphate-mediated DNA transfection, Wigler, et al., Cell 11:223 (1977). This plasmid-containing cells are referred to as a "packaging cell line." These plasmid containing packaging cell lines can be maintained as such or a replication incompetent retroviral vector can be introduced into the cell's genome. In the latter case, the genomic RNA generated by the vector construct combines with the constitutively expressed retroviral structural proteins of the packaging line, resulting in the release of retroviral particles into the culture medium. A stable cell line containing the structural gene sequences of the retroviruses is a retroviral "producer cell line."

Because genes can be introduced into MSCs, MAPCs, and/or other stem cells using a retroviral vector, they can be "on" (subject to) the retroviral vector control; in such a case, the gene of interest is transcribed from a retroviral promoter. A promoter is a specific nucleotide sequence recognized by RNA polymerase molecules that start RNA synthesis. Alternatively, retroviral vectors having additional promoter elements (in addition to the promoter incorporated in the recombinant retrovirus), which are responsible for the transcription of the genetic material of interest, can be used. For example, a construct in which there is an additional promoter modulated by an external factor or cue can be used, making it possible to control the level of polypeptides being produced by the MSCs, MAPCs, and/or other stem cells by activating that external factor of cue. For example, heat shock proteins are proteins encoded by genes in which the promoter is regulated by temperature. The promoter of the gene, which encodes the metal- containing protein metallothionine, is responsive to cadmium (Cd⁺⁺) ions. Incorporation of this promoter or another promoter influenced by external cues also makes it possible to regulate the production of the polypeptide by the engineered progenitor cells.

Examples of vectors other than retroviruses that can be used to genetically engineer or modify MSCs, MAPCs, and/or other stem cells can be derived from adenovirus (Ad) or adeno-associated virus (AAV). Both human and non-human viral vectors can be used but preferably the recombinant viral vector is replication-defective in humans. Where the vector is an adenovirus, it preferably comprises a polynucleotide having a promoter operably linked to a gene encoding the CXCR4 and is replication-defective in humans.

Adenovirus vectors are capable of highly efficient gene expression in target cells and can accommodate a relatively large amount of heterologous (non-viral) DNA. A preferred form of recombinant adenovirus is a "gutless, "high-capacity", or "helper-dependent" adenovirus vector. Such a vector features, for example, (1) the deletion of all or most viral-coding sequences (those sequences encoding viral proteins), (2) the viral inverted terminal repeats (ITRs) which are sequences required for viral DNA replication, (3) up to 28-32 kb of "exogenous" or "heterologous" sequences (*e.g*., sequences encoding CXCR4), and (4) the viral DNA packaging sequence which is required for packaging of the viral genomes into infectious capsids. For specifically myocardial cells, preferred variants of such recombinant adenoviral vectors contain tissue-specific (*e.g*., MSCs, MAPCs, and/or other stem cells) enhancers and promoters operably linked to a CXCR4 gene.

AAV-based vectors are advantageous because they exhibit high transduction efficiency of target cells and can integrate into the target genome in a site-specific manner. Use of recombinant AAV vectors is discussed in detail in Tal, J., J. Biomed. Sci. 7:279-291, 2000 and Monahan and Samulski, Gene Therapy 7:24-30, 2000. A preferred AAV vector comprises a pair of AAV inverted terminal repeats which flank at least one cassette containing a tissue or cell specific promoter operably linked to a CXCR4 nucleic acid. The DNA sequence of the AAV vector, including the ITRs, the promoter and CXCR4 gene may be integrated into the target genome.

Other viral vectors that can be use in accordance with the present invention include herpes simplex virus (HSV)-based vectors. HSV vectors deleted of one or more immediate early genes (IE) are advantageous because they are generally non-cytotoxic, persist in a state similar to latency in the target cell, and afford efficient target cell transduction. Recombinant HSV vectors can incorporate approximately 30 kb of heterologous nucleic acid. A preferred HSV vector is one that: (1) is engineered from HSV type I, (2) has its IE genes deleted, and (3) contains a tissue-specific (*e.g*., myocardium) promoter operably linked to a SDF-1 nucleic acid. HSV amplicon vectors may also be useful in various methods of the invention. Typically, HSV amplicon vectors are approximately 15 kb in length, and possess a viral origin of replication and packaging sequences.

Alphavirus-based vectors, such as those made from semliki forest virus (SFV) and sindbis virus (SIN), might also be used in the invention. Use of alphaviruses is described in Lundstrom, K., Intervirology 43:247-257, 2000 and Perri et al., Journal of Virology 74:9802-9807, 2000. Alphavirus vectors typically are constructed in a format known as a replicon. A replicon may contain (1) alphavirus genetic elements required for RNA replication, and (2) a heterologous nucleic acid such as one encoding a CXCR4 nucleic acid. Within an alphavirus replicon, the heterologous nucleic acid may be operably linked to a tissue-specific promoter or enhancer.

Recombinant, replication-defective alphavirus vectors are advantageous because they are capable of high-level heterologous (therapeutic) gene expression, and can infect a wide target cell range. Alphavirus replicons may be targeted to specific cell types (*e.g*., MSCs, MAPCs, and/or other stem cells) by displaying on their virion surface a functional heterologous ligand or binding domain that would allow selective binding to target cells expressing a cognate binding partner. Alphavirus replicons may establish latency, and therefore long-term heterologous nucleic acid expression in a target cell. The replicons may also exhibit transient heterologous nucleic acid expression in the target cell. A preferred alphavirus vector or replicon is non-cytopathic.

In many of the viral vectors compatible with methods of the invention, more than one promoter can be included in the vector to allow more than one heterologous gene to be expressed by the vector. Further, the vector can comprise a sequence, which encodes a signal peptide, or other moiety, which facilitates the expression of a CXCR4 gene product from the target cell.

To combine advantageous properties of two viral vector systems, hybrid viral vectors may be used to deliver a CXCR4 nucleic acid to the MSCs, MAPCs, and/or other stem cells. Standard techniques for the construction of hybrid vectors are well-known to those skilled in the art. Such techniques can be found, for example, in Sambrook, *et al.,* In Molecular Cloning: A laboratory manual. Cold Spring Harbor, N.Y. or any number of laboratory manuals that discuss recombinant DNA technology. Double-stranded AAV genomes in adenoviral capsids containing a combination of AAV and adenoviral ITRs may be used to transduce cells. In another variation, an AAV vector may be placed into a "gutless", "helper-dependent" or "high-capacity" adenoviral vector. Adenovirus/AAV hybrid vectors are discussed in Lieber et al., J. Virol. 73:9314-9324, 1999. Retrovirus/adenovirus hybrid vectors are discussed in Zheng et al., Nature Biotechnol. 18:176-186, 2000. Retroviral genomes contained within an adenovirus may integrate within the target cell genome and effect stable CXCR4 gene expression.

Other nucleotide sequence elements, which facilitate expression of the CXCR4 gene and cloning of the vector, are further contemplated. For example, the presence of enhancers upstream of the promoter or terminators downstream of the coding region, for example, can facilitate expression.

In addition to viral vector-based methods, non-viral methods may also be used to introduce a CXCR4 gene into a target cell. A review of non-viral methods of gene delivery is provided in Nishikawa and Huang, Human Gene Ther. 12:861-870, 2001. A preferred non-viral gene delivery method according to the invention employs plasmid DNA to introduce a CXCR4 nucleic acid into a cell. Plasmid-based gene delivery methods are generally known in the art.

Synthetic gene transfer molecules can be designed to form multimolecular aggregates with plasmid DNA. These aggregates can be designed to bind to the MSCs, MAPCs, and/or other stem cells.

Cationic amphiphiles, including lipopolyamines and cationic lipids, may be used to provide receptor-independent CXCR4 nucleic acid transfer into MSCs, MAPCs, and/or other stem cells. In addition, preformed cationic liposomes or cationic lipids may be mixed with plasmid DNA to generate cell-transfecting complexes. Methods involving cationic lipid formulations are reviewed in Felgner et al., Ann. N.Y. Acad. Sci. 772:126-139, 1995 and Lasic and Templeton, Adv. Drug Delivery Rev. 20:221-266, 1996. For gene delivery, DNA may also be coupled to an amphipathic cationic peptide (Fominaya et al., J. Gene Med. 2:455-464, 2000).

Methods that involve both viral and non-viral based components may be used according to the invention. For example, an Epstein Barr virus (EBV)-based plasmid for therapeutic gene delivery is described in Cui et al., Gene Therapy 8:1508-1513, 2001. Additionally, a method involving a DNA/ligand/polycationic adjunct coupled to an adenovirus is described in Curiel, D. T., Nat. Immun. 13:141-164, 1994.

Vectors containing nucleic acids that encode the expression of CXCR4 can be delivered to the MSCs, MAPCs, and/or other stem cells that are cultured *ex vivo* or *in vitro* by direct injection into the culture medium. The injectable preparation can contain pharmaceutically acceptable carrier, such as saline, as necessary. Other pharmaceutical carriers, formulations and dosages can also be used in accordance with the present invention.

The MSCs, MAPCs, and/or other stem cells genetically modified to over-express CXCR4 when introduced into a mammalian subject for therapeutic applications and/or cellular therapy have improved survivability and longevity compared to MSCs, which are not genetically modified to over-express CXCR4. The over-expressed CXCR4 of the genetically modified MSCs, MAPCs, and/or other stem can bind to SDF-1 chemokine that is present in the tissue to be treated. Binding of SDF-1 to CXCR4 can induce phosphorylation of protein kinase AKT, which can in turn mitigate apoptosis and substantially improve the survival of the MSCs, MAPCs, and/or other stem cells.

### SDF-1 over-expression

In accordance with another aspect of the invention, the MSCs, MAPCs, and/or other stem cells can be genetically modified to over-express stromal cell derived factor 1 (SDF-1). SDF-1, also known as CXC chemokine L12, is a member of the CXC family of chemokines and is thought to be the natural ligand of CXCR4. SDF-1 is functionally distinct from other chemokines in that it is reported to have a fundamental role in the trafficking, export, and homing of bone marrow progenitor cells. SDF-1 is also structurally distinct in that it has only about 22% amino acid sequence identity with other CXC chemokines. A basic physiological role of SDF-1 is implied by a high conservation of the SDF-1 sequence between species. *In vitro,* SDF-1 stimulates chemotaxis of a wide range of cells including monocytes and bone marrow derived progenitor cells.

The amino acid sequence of a number of different mammalian SDF-1 protein are known including human, mouse, and rat. The human and rat SDF-1 amino acid sequences are about 92% identical. SDF-1 can comprise two isoform, SDF-1 alpha and SDF-1 beta, both of which are referred to herein as SDF-1 unless identified otherwise.

The SDF-1 that is over-expressed can have an amino acid sequence substantially identical to SEQ ID NO: 5. The SDF-1 that is over-expressed can also have an amino acid sequence substantially similar to one of the foregoing mammalian SDF-1 proteins. For example, the SDF-1 that is over-expressed can have an amino acid sequence substantially similar to SEQ ID NO: 6. SEQ ID NO: 6, which substantially comprises SEQ ID NO: 5, is the amino sequence for human SDF and is identified by GenBank Accession No. NP954637. The SDF-1 that is over-expressed can also have an amino acid sequence that is substantially identical to SEQ ID NO: 7. SEQ ID NO: 7, which also substantially comprises SEQ ID NO: 5, includes the amino acid sequences for rat SDF and is identified by GenBank Accession No. AAF01066.

The SDF-1 that is over-expressed in accordance with the present invention can also be a variant of mammalian SDF-1, such as a fragment, analog and derivative of mammalian SDF-1. Such variants include, for example, a polypeptide encoded by a naturally occurring allelic variant of native SDF-1 gene *(i.e.,* a naturally occurring nucleic acid that encodes a naturally occurring mammalian SDF-1 polypeptide), a polypeptide encoded by an alternative splice form of a native SDF-1 gene, a polypeptide encoded by a homolog or ortholog of a native SDF-1 gene, and a polypeptide encoded by a non-naturally occurring variant of a native SDF-1 gene.

SDF-1 variants have a peptide sequence that differs from a native SDF-1 polypeptide in one or more amino acids. The peptide sequence of such variants can feature a deletion, addition, or substitution of one or more amino acids of a SDF-1 variant. Amino acid insertions are preferably of about 1 to 4 contiguous amino acids, and deletions are preferably of about 1 to 10 contiguous amino acids. Variant SDF-1 polypeptides substantially maintain a native SDF-1 functional activity. Preferred SDF-1 polypeptide variants can be made by expressing nucleic acid molecules within the invention that feature silent or conservative changes.

SDF-1 polypeptide fragments corresponding to one or more particular motifs and/or domains or to arbitrary sizes, are within the scope of the present invention. Isolated peptidyl portions of SDF-1 can be obtained by screening peptides recombinantly produced from the corresponding fragment of the nucleic acid encoding such peptides. For example, a SDF-1 polypeptides of the present invention may be arbitrarily divided into fragments of desired length with no overlap of the fragments, or preferably divided into overlapping fragments of a desired length. The fragments can be produced recombinantly and tested to identify those peptidyl fragments, which can function as agonists of native CXCR-4 polypeptides.

Variants of SDF-1 polypeptides can also include recombinant forms of the SDF-1 polypeptides. Recombinant polypeptides preferred by the present invention, in addition to SDF-1 polypeptides, are encoded by a nucleic acid that can have at least 70% sequence identity with the nucleic acid sequence of a gene encoding a mammalian SDF-1.

SDF-1 variants can include agonistic forms of the protein that constitutively express the functional activities of native SDF-1. Other SDF-1 variants can include those that are resistant to proteolytic cleavage, as for example, due to mutations, which alter protease target sequences. Whether a change in the amino acid sequence of a peptide results in a variant having one or more functional activities of a native SDF-1 can be readily determined by testing the variant for a native SDF-1 functional activity.

The MSCs, MAPCs, and/or other stem cells in accordance with the present invention can be genetically modified with a nucleic acid that encodes SDF-1 or a variant of SDF-1. The nucleic acid can be a native or non-native nucleic acid and be in the form of RNA or in the form of DNA (*e.g*., cDNA, genomic DNA, and synthetic DNA). The DNA can be double-stranded or single-stranded, and if single-stranded may be the coding (sense) strand or non-coding (anti-sense) strand. The nucleic acid coding sequence that encodes SDF-1 may be substantially similar to a nucleotide sequence of the SDF-1 gene, such as nucleotide sequence shown in SEQ ID NO: 8 and SEQ ID NO: 9. SEQ ID NO: 8 and SEQ ID NO: 9 comprise, respectively, the nucleic acid sequences for human SDF-1 and rat SDF-1 and are substantially similar to the nucleic sequences of GenBank Accession No. NM199168 and GenBank Accession No. AF189724. The nucleic acid coding sequence for SDF-1 can also be a different coding sequence which, as a result of the redundancy or degeneracy of the genetic code, encodes the same polypeptide as SEQ ID NO: 5, SEQ ID NO: 6, and SEQ ID NO: 7.

Other nucleic acid molecules that encode SDF-1 within the invention are variants of a native SDF-1, such as those that encode fragments, analogs and derivatives of native SDF-1. Such variants may be, for example, a naturally occurring allelic variant of a native SDF-1 gene, a homolog or ortholog of a native SDF-1 gene, or a non-naturally occurring variant of a native SDF-1 gene. These variants have a nucleotide sequence that differs from a native SDF-1 gene in one or more bases. For example, the nucleotide sequence of such variants can feature a deletion, addition, or substitution of one or more nucleotides of a native SDF-1 gene. Nucleic acid insertions are preferably of about 1 to 10 contiguous nucleotides, and deletions are preferably of about 1 to 10 contiguous nucleotides.

In other applications, variant SDF-1 displaying substantial changes in structure can be generated by making nucleotide substitutions that cause less than conservative changes in the encoded polypeptide. Examples of such nucleotide substitutions are those that cause changes in (a) the structure of the polypeptide backbone; (b) the charge or hydrophobicity of the polypeptide; or (c) the bulk of an amino acid side chain. Nucleotide substitutions generally expected to produce the greatest changes in protein properties are those that cause non-conservative changes in codons. Examples of codon changes that are likely to cause major changes in protein structure are those that cause substitution of (a) a hydrophilic residue(*e.g*., serine or threonine), for (or by) a hydrophobic residue (*e.g*., leucine, isoleucine, phenylalanine, valine or alanine); (b) a cysteine or proline for (or by) any other residue; (c) a residue having an electropositive side chain (*e.g*., lysine, arginine, or histidine), for (or by) an electronegative residue (*e.g*., glutamine or aspartine); or (d) a residue having a bulky side chain (*e.g*., phenylalanine), for (or by) one not having a side chain, (*e.g*., glycine).

Naturally occurring allelic variants of a native SDF-1 gene within the invention are nucleic acids isolated from mammalian tissue that have at least 70% sequence identity with a native SDF-1 gene, and encode polypeptides having structural similarity to a native SDF-1 polypeptide. Homologs of a native SDF-1 gene within the invention are nucleic acids isolated from other species that have at least 70% sequence identity with the native gene, and encode polypeptides having structural similarity to a native SDF-1 polypeptide. Public and/or proprietary nucleic acid databases can be searched to identify other nucleic acid molecules having a high percent (*e.g*., 70% or more) sequence identity to a native SDF-1 gene.

Non-naturally occurring SDF-1 gene variants are nucleic acids that do not occur in nature (*e.g*., are made by the hand of man), have at least 70% sequence identity with a native SDF-1 gene, and encode polypeptides having structural similarity to a native SDF-1 polypeptide. Examples of non-naturally occurring SDF-1 gene variants are those that encode a fragment of a native SDF-1 protein, those that hybridize to a native SDF-1 gene or a complement of to a native SDF-1 , gene under stringent conditions, and those that share at least 65% sequence identity with a native SDF-1 gene or a complement of a native SDF-1 gene.

Nucleic acids encoding fragments of a native SDF-1 gene within the invention are those that encode, amino acid residues of native SDF-1. Shorter oligonucleotides that encode or hybridize with nucleic acids that encode fragments of native SDF-1 can be used as probes, primers, or antisense molecules. Longer polynucleotides that encode or hybridize with nucleic acids that encode fragments of a native SDF-1 can also be used in various aspects of the invention. Nucleic acids encoding fragments of a native SDF-1 can be made by enzymatic digestion (*e.g*., using a restriction enzyme) or chemical degradation of the full length native SDF-1 gene or variants thereof.

Nucleic acids that hybridize under stringent conditions to one of the foregoing nucleic acids can also be used in the invention. For example, such nucleic acids can be those that hybridize to one of the foregoing nucleic acids under low stringency conditions, moderate stringency conditions, or high stringency conditions are within the invention.

Nucleic acid molecules encoding a SDF-1 fusion protein may also be used in the invention. Such nucleic acids can be made by preparing a construct (*e.g*., an expression vector) that expresses a SDF-1 fusion protein when introduced into a suitable target cell. For example, such a construct can be made by ligating a first polynucleotide encoding a SDF-1 protein fused in frame with a second polynucleotide encoding another protein such that expression of the construct in a suitable expression system yields a fusion protein.

The nucleic acids used over-express SDF-1 can be modified at the base moiety, sugar moiety, or phosphate backbone, for example, to improve stability of the molecule, hybridization, etc. The nucleic acids within the invention may additionally include other appended groups such as peptides (*e.g*., for targeting target cell receptors *in vivo*)*,* or agents facilitating transport across the cell membrane, hybridization-triggered cleavage. To this end, the nucleic acids may be conjugated to another molecule, (*e.g*., a peptide), hybridization triggered crosslinking agent, transport agent, hybridization-triggered cleavage agent, etc.

The SDF-1 can be over-expressed from the MSCs, MAPCs, and/or other stem cells by introducing an agent into the stem cells that promotes expression of SDF-1. The agent can comprise natural or synthetic nucleic acids, according to present invention and described above, that are incorporated into recombinant nucleic acid constructs, typically DNA constructs, capable of introduction into and replication in the cell. Such a construct preferably includes a replication system and sequences that are capable of transcription and translation of a polypeptide-encoding sequence in a given target cell.

Other agents can also be introduced into the MSCs, MAPCs, and/or other stem cells to promote expression of SDF-1 from the stem cells. For example, agents that increase the transcription of a gene encoding SDF-1, increase the translation of an mRNA encoding SDF-1, and/ or those that decrease the degradation of an mRNA encoding SDF-1 could be used to increase SDF-1 protein levels. Increasing the rate of transcription from a gene within a cell can be accomplished by introducing an exogenous promoter upstream of the gene encoding SDF-1. Enhancer elements, which facilitate expression of a heterologous gene, may also be employed.

A preferred method of introducing the agent into a MSCs, MAPCs, and/or other stem cells involves using gene therapy. One method of gene therapy uses a vector including a nucleotide encoding SDF-1. Vectors include, for example, viral vectors (such as adenoviruses ('Ad'), adeno-associated viruses (AAV), and retroviruses), liposomes and other lipid-containing complexes, and other macromolecular complexes capable of mediating delivery of a polynucleotide to a target cell (*i.e*., MSCs, MAPCs, and/or other stem cells).

Vectors can also comprise other components or functionalities that further modulate gene delivery and/or gene expression, or that otherwise provide beneficial properties to the targeted cells. Such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors, which have components or functionalities mediating binding and uptake), or vectors can be modified to provide such functionalities.

Selectable markers can be positive, negative or bifunctional. Positive selectable markers allow selection for cells carrying the marker, whereas negative selectable markers allow cells carrying the marker to be selectively eliminated. A variety of such marker genes have been described, including bifunctional (*i.e*. positive/negative) markers (see, *e.g*., Lupton, S., WO 92/08796, published May 29, 1992; and Lupton, S., WO 94/28143, published Dec. 8, 1994). Such marker genes can provide an added measure of control that can be advantageous in gene therapy contexts. A large variety of such vectors are known in the art and are generally available.

Vectors for use in the present invention include viral vectors, lipid based vectors and other vectors that are capable of delivering a nucleotide according to the present invention to the MSCs, MAPCs, and/or other stem cells. The vector can be a targeted vector, especially a targeted vector that preferentially binds to MSCs, MAPCs, and/or other stem cells. Preferred viral vectors for use in the invention are those that exhibit low toxicity to a target cell and induce production of therapeutically useful quantities of SDF-1.

One example of a viral vector that can be used to genetically modify MSCs, MAPCs, and/or other stem cells used to regenerate myocardial structures is a retrovirus. Other examples of vectors that can also be used to genetically engineer or modify stem cells can be derived from adenovirus (Ad) or adeno-associated virus (AAV). Still other viral vectors and non-viral vectors well known in the art and described above can be used.

The MSCs, MAPCs, and/or other stem cells genetically modified to over-express SDF-1 when introduced into a mammalian subject for therapeutic applications and/or cellular therapy have improved,survivability and longevity compared to MSCs, which are not genetically modified to over-express SDF-1. The over-expressed SDF-1 of the genetically modified MSCs, MAPCs, and/or other stem cells can bind to CXCR4 that is present in the MSCs, MAPCs and/or other stem cells as well as the CXCR4 that is present in the tissue to be treated. Binding of SDF-1 to CXCR4 can induce phosphorylation of protein kinase AKT, which can in turn mitigate apoptosis and substantially improve the survival of the MSCs, MAPCs, and/or other stem cells and the tissue to be treated. Additionally, the over-expression of SDF-1 can promote homing of the MSCs, MAPCs, and/or other stem cells used to regenerate myocardial structures as well as the homing of additional progenitor or stem cells in the mammalian subject to the tissue being treated.

### CXCR4 and SDF-1 over-expression

In accordance with yet another aspect of the invention, the MSCs, MAPCs, and/or other stem cells can be genetically modified to over-express both CXCR4 and SDF-1. The CXCR4 polypeptide that is over-expressed in accordance with the present can have an amino acid sequence substantially similar to the amino acid sequence of mammalian CXCR4 polypeptides. For example, the CXCR4 that is over-expressed can have an amino acid sequence substantially similar to SEQ ID NO: 1 and/or SEQ ID NO: 2. The SDF-1 that is over-expressed can also have an amino acid sequence substantially identical to the amino acid sequence of mammalian SDF-1. For example, the SDF-1 that is over-expressed can have an amino acid sequence substantially identical to SEQ ID NO: 5, SEQ ID NO: 6, and/or SEQ ID NO: 7.

The CXCR4 and SDF-1 that are over-expressed in accordance with the present invention can also be a variant of, respectively, mammalian CXCR4 and mammalian SDF-1, such as a fragment, analog and derivative of, respectively mammalian CXCR4 and mammalian SDF-1. Such variants include, for example, a polypeptide encoded by a naturally occurring allelic variant of native CXCR4 gene *(i.e.,* a naturally occurring nucleic acid that encodes a naturally occurring mammalian CXCR4 polypeptide), a polypeptide encoded by a naturally occurring allelic variant of native SDF-1 gene, a polypeptide encoded by an alternative splice form of a native CXCR4 gene, a polypeptide encoded by an alternative splice form of a native SDF-1 gene, a polypeptide encoded by a homolog or ortholog of a native CXCR4 gene, a polypeptide encoded by a homolog or ortholog of a native SDF-1 gene, a polypeptide encoded by a non-naturally occurring variant of a native CXCR4 gene, and/or a polypeptide encoded by a non-naturally occurring variant of a native SDF-1 gene.

CXCR4 and SDF-1 variants have a peptide sequence that differs from a native CXCR4 and native SDF-1 in one or more amino acids. The peptide sequence of such variants can feature a deletion, addition, or substitution of one or more amino acids of a CXCR4 variant and/or a SDF-1 variant. Amino acid insertions are preferably of about 1 to 4 contiguous amino acids, and deletions are preferably of about 1 to 10 contiguous amino acids. Variant CXCR4 polypeptides substantially maintain native CXCR4 functional activity, while variant SDF-1 polypeptides substantially maintain a native SDF-1 functional activity.

CXCR4 and SDF-1 polypeptide fragments corresponding to one or more particular motifs and/or domains or to arbitrary sizes, are within the scope of the present invention. Variants of CXCR4 and SDF-1 polypeptides can also include recombinant forms of CXCR4 and SDF-1 polypeptides. Recombinant polypeptides preferred by the present invention are encoded by a nucleic acid that can have at least 70% sequence identity with the nucleic acid sequence of genes encoding, respectively, mammalian CXCR4 and mammalian SDF-1.

CXCR4 and SDF-1 variants can include agonistic forms of the protein that constitutively express the functional activities of, respectively, native CXCR4 and SDF-1. Other CXCR4 and SDF-1 variants can include those that are resistant to proteolytic cleavage, as for example, due to mutations, which alter protease target sequences.

Over-expression of CXCR4 and SDF-1 from the MSCs, MAPCs, and/or other stem cells used to regenerate myocardial structures can be performed by genetically modifying the stem cells with a nucleic acids that encode CXCR4 (or a variant of CXCR4) and SDF-1 (or a variant of SDF-1). The nucleic acids can be a native or non-native nucleic acid and be in the form of RNA or in the form of DNA (*e.g*., cDNA, genomic DNA, and synthetic DNA). The DNA can be double-stranded or single-stranded, and if single-stranded may be the coding (sense) strand or non-coding (anti-sense) strand.

The nucleic acid coding sequence that encodes a CXCR4 polypeptide may be substantially similar to nucleotide sequence shown in SEQ ID NO: 3 and SEQ ID NO: 4. The nucleic acid coding sequence that CXCR4 can also be a different coding sequence which, as a result of the redundancy or degeneracy of the genetic code, encodes the same polypeptide as SEQ ID NO: 3 and SEQ ID NO: 4.

The nucleic acid coding sequence that encodes SDF-1 may be substantially similar to a nucleotide sequence shown in SEQ ID NO: 8 and SEQ NO: 9. The nucleic acid coding sequence for SDF-1 can also be a different coding sequence which, as a result of the redundancy or degeneracy of the genetic code, encodes the same polypeptide as SEQ ID NO: 5, SEQ ID NO: 6, and SEQ ID NO: 7.

Other nucleic acid molecules that encode CXCR4 and SDF-1 within the invention are, respectively, variants of native CXCR4 and of native SDF-1, such as those that encode fragments, analogs and derivatives of native CXCR4 and native SDF-1. These variants can have a nucleotide sequence that differs, respectively, from a native CXCR4 gene and SDF-1 gene in one or more bases.

In other applications, variant CXCR4 and variant SDF-1 displaying substantial changes in structure can be generated by making nucleotide substitutions that cause less than conservative changes in the encoded polypeptide. Naturally occurring allelic variants of a native CXCR4 gene and a native SDF-1 gene within the invention are nucleic acids isolated from mammalian tissue that have at least 70% sequence identity with, respectively, a native CXCR4 gene and a native SDF-1 gene, and encode polypeptides having structural similarity to a CXCR polypeptide and a native SDF-1 polypeptide.

Nucleic acids that hybridize under stringent conditions to one of the foregoing nucleic acids can also be used in the invention. For example, such nucleic acids can be those that hybridize to one of the foregoing nucleic acids under low stringency conditions, moderate stringency conditions, or high stringency conditions are within the invention.

Nucleic acid molecules encoding a CXCR4 and SDF-1 fusion protein may also be used in the invention. Such nucleic acids can be made by preparing a construct (*e.g*., an expression vector) that expresses a CXCR4 and SDF-1 fusion protein when introduced into a suitable target cell.

The nucleic acids used over-express CXCR4 and SDF-1 can be modified at the base moiety, sugar moiety, or phosphate backbone, for example, to improve stability of the molecule, hybridization, etc. The nucleic acids within the invention may additionally include other appended groups such as peptides (*e.g*., for targeting target cell receptors *in vivo*)*,* or agents facilitating transport across the cell membrane, hybridization-triggered cleavage. To this end, the nucleic acids may be conjugated to another molecule, *e.g*., a peptide, hybridization triggered crosslinking agent, transport agent, hybridization-triggered cleavage agent, etc.

The CXCR4 and SDF-1 can be over-expressed from the MSCs, MAPCs, and/or other stem cells by introducing at least one agent into the MSCs, MAPCs, and/or other stem cells that promotes expression of CXCR4 and SDF-1. The agent can comprise natural or synthetic nucleic acids, according to present invention and described above, that are incorporated into recombinant nucleic acid constructs, typically DNA constructs, capable of introduction into and replication in the cell. Such a construct preferably includes a replication system and sequences that are capable of transcription and translation of a polypeptide-encoding sequence in a given target cell.

Other agents can also be introduced into the MSCs, MAPCs, and/or other stem cells to promote expression of CXCR4 and SDF-1 from the stem cells. For example, agents that increase the transcription of the genes encoding CXCR4 and SDF-1, increase the translation of mRNA encoding CXCR4 and SDF-1, and/ or those that decrease the degradation of an mRNA encoding CXCR4 and SDF-1 could be used to over-express CXCR4 and SDF-1. Increasing the rate of transcription from a gene within a cell can be accomplished by introducing an exogenous promoter upstream of the gene encoding CXCR4 and of the gene encoding SDF-1. Enhancer elements that facilitate expression of a heterologous gene may also be employed.

A preferred method of introducing the agent into MSCs, MAPCs, and/or other stem cells involves using gene therapy. One method of gene therapy uses a vector including a nucleotide encoding CXCR4 and a vector including a nucleotide encoding SDF-1. Another method uses a vector encoding both CXCR4 and SDF-1. Vectors include, for example, viral vectors (such as adenoviruses ('Ad'), adeno-associated viruses (AAV), and retroviruses), liposomes and other lipid-containing complexes, and other macromolecular complexes capable of mediating delivery of a polynucleotide to a target cell *(i.e.,* MSCs, MAPCs, and/or other stem cells).

Vectors can also comprise other components or functionalities that further modulate gene delivery and/or gene expression, or that otherwise provide beneficial properties to the targeted cells. Such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors, which have components or functionalities mediating binding and uptake), or vectors can be modified to provide such functionalities.

Selectable markers can be positive, negative or bifunctional. Positive selectable markers allow selection for cells carrying the marker, whereas negative selectable markers allow cells carrying the marker to be selectively eliminated. A variety of such marker genes have been described, including bifunctional (*i.e*. positive/negative) markers (see, *e.g*., Lupton, S., WO 92/08796, published May 29, 1992; and Lupton, S., WO 94/28143, published Dec. 8, 1994). Such marker genes can provide an added measure of control that can be advantageous in gene therapy contexts. A large variety of such vectors are known in the art and are generally available.

Vectors for use in the present invention include viral vectors, lipid based vectors and other vectors that are capable of delivering a nucleotide according to the present invention to the MSCs, MAPCs, and/or other stem cells. The vector can be a targeted vector, especially a targeted vector that preferentially binds to MSCs, MAPCs, and/or other stem cells. Preferred viral vectors for use in the invention are those that exhibit low toxicity to a target cell and induce production of therapeutically useful quantities of CXR4 and SDF-1.

One example of a viral vector that can be used to genetically modify MSCs, MAPCs, and/or other stem cells is a retrovirus. Other examples of vectors that can also be used to genetically engineer or modify the stem cells can be derived from adenovirus (Ad) or adeno-associated virus (AAV). Still other viral vectors and non-viral vectors well known in the art and described above can be used.

The MSCs, MAPCs, and/or other stem cells genetically modified to over-express both CXCR4 and SDF-1 when introduced into a mammalian subject for therapeutic applications and/or cellular therapy have improved survivability and longevity compared to MSCs, MAPCs, and/or other stem cells, which are not genetically modified to over-express either CXCR4 and/or SDF-1. The over-expressed CXCR4 and SDF-1 of the genetically modified MSCs, MAPCs, and/or other stem cells used to regenerate myocardial structures can bind to together in the stem cells |as well as bind to CXCR4 and SDF-1 that is present in the tissue to be treated. As discussed previously, binding of SDF-1 to CXCR4 can induce phosphorylation of protein kinase AKT, which can in turn mitigate apoptosis and substantially improve the survival of the MSCs, MAPCs, and/or other stem cells and the tissue to be treated. Additionally, the over-expression of CXCR4 and SDF-1 can promote homing of the MSCs, MAPCs, and/or other stem cells used to regenerate myocardial structures as well as the homing of additional progenitor or stem cells in the mammalian subject to the tissue being treated.

### Therapeutic applications

The MSCs, MAPCs, and/or other stem cells genetically modified to over-express CXCR4, SDF-1, or both CXCR4 and SDF-1 can be used for potentially any cellular therapy or therapeutic application where it is desirable to expand, repopulate, preserve, and/or regenerate tissue and/or organ systems. In accordance with an aspect of the present invention, the MSCs, MAPCs, and/or other stem cells genetically modified to over-express CXCR4 can be used to treat patients with recent myocardial infarction or congestive heart failure. Patients with recent myocardial infarction or congestive heart failure can be treated by delivering the genetically modified MSCs, MAPCs, and/or other stem cells to the infarcted myocardial tissue and/or to tissue proximate the infarcted myocardial tissue. The genetically modified MSCs, MAPCs, and/or other stem cells delivered to the infarcted myocardial tissue can differentiate into cells, which can repopulate (*i.e*., engraft) and partially or wholly restore the normal function of the infarcted myocardium.

The genetically modified MSCs, MAPCs, and/or other stem cells used to regenerate myocardial structures can be delivered to the infarcted myocardium by directly injecting the genetically modified MSCs, MAPCs, and/or other stem cells used to into the infarcted myocardial tissue or myocardial tissue proximate the infarction. Direct injection of the genetically modified stem cells can be performed by using, for example, a tuberculin syringe. Direct injection of the genetically modified stem cells into the infarcted myocardial tissue can upregulate the expression of SDF-1 from the infarcted myocardial tissue. The up-regulation of SDF-1 expression in the infarcted myocardial tissue has been observed from about 1 hour after transplantation of the MSCs, MAPCs, and/or other stem cells into the myocardium to less than seven days after transplantation. This up-regulation of SDF-1 can potentially cause pluripotent stem cells in the peripheral blood to be homed to the infarcted myocardium. The over-expression of CXCR4 and/or SDF-1 from the MSCs, MAPCs, and/or other stem cells enhances the survivability of the genetically modified stem cells, which increases therapeutic efficacy of the treatment.

Alternatively, the genetically modified MSCs, MAPCs, and/or other stem cells used to regenerate myocardial structures can be delivered to the infarcted myocardial tissue by venous or arterial infusion of the genetically modified stem cells into the mammalian subject to be treated. The infusion of the MSCs, MAPCs, and/or other stem cells used to regenerate myocardial structures over-expressing SDF-1 and/or CXCR4 can be performed soon (*e.g*., about 1 day) after the myocardial infarction. A myocardial infarction cause a temporal up-regulation in SDF-1 expression in the infarcted myocardial tissue. This up-regulation of SDF-1 expression can potentially cause the genetically modified MSCs, MAPCs, and/or other stem cells used to regenerate myocardial structures that are infused into the peripheral blood of the mammalian subject to home to the infarcted myocardial tissue. The over-expression of CXCR4 and/or SDF-1 from the MSCs, MAPCs, and/or other stem cells used to regenerate myocardial structures enhances the survivability of the genetically modified stem cells, which increases therapeutic efficacy of the treatment.

MSCs, MAPCs, and/or other stem cells used to regenerate myocardial structures once delivered to the tissue to be treated can express the CXCR4 and/or SDF-1 for any suitable length of time, including transient expression and stable, long-term expression. In a preferred embodiment, the CXCR4 and/or SDF-1 will be expressed in therapeutic amounts for a suitable and defined length of time.

A therapeutic amount is an amount, which is capable of producing a medically desirable result in a treated animal or human. As is well known in the medical arts, dosage for any one animal or human depends on many factors, including the subject's size, body surface area, age, the particular composition to be administered, sex, time and route of administration, general health, and other drugs being administered concurrently. Specific dosages of proteins, nucleic acids, or small molecules) can be determined readily determined by one skilled in the art using the experimental methods described below.

Long term CXCR4 and/or SDF-1 expression from the MSCs, MAPCs, and/or other stem cells used to regenerate myocardial structures is advantageous because it allows for the administration of a mobilizing agent at a time remote (e.g., about two to three days post myocardial infarction) from the delivery of the genetically modified MSCs, MAPCs, and/or other stem cells used to regenerate myocardial structures. Administration, of the mobilizing agent can induce mobilization of other stem cells from tissue (e.g., bone marrow) to the peripheral blood of the subject and increase stem cell concentration within the peripheral blood. In the case where granulocyte-colony stimulating factor (G-CSF) is the mobilizing agent there is a significant increase in neutrophil count, which could cause negative effects in the peri-surgical period, but not days or weeks later. Additionally, long term or chronic over-expression of CXCR4 and/or SDF-1 causes long term homing of stem cells into the infarcted myocardial tissue from the peripheral blood without the need of stem cell mobilization.

It will be appreciated that a number of other mobilizing agents are known and can be used in accordance with the present invention. The agents can include cytokines, such as, granulocyte-macrophage colony stimulating factor (GM-CSF), interleukin (IL)-7, IL-3, IL-12, stem cell factor (SCF), and flt-3 ligand; chemokines such as IL-8, Mip-1α, and Groβ, and the chemotherapeutic agents of cylcophosamide (Cy) and paclitaxel. These agents differ in their time frame to achieve stem cell mobilization, the type of stem cell mobilized, and efficiency. One skilled in the art will appreciate that yet other mobilizing agents can also be administered.

The mobilizing agent can be administered by direct injection of the mobilizing agent into the subject. Although, the mobilizing agent is typically administered after the genetically modified MSCs, MAPCs, and/or other stem cells used to regenerate myocardial structures are delivered (e.g., direct injected or infuse) to tissue to be treated, the mobilizing agent, however, can be administered before the genetically modified stem cells are delivered.

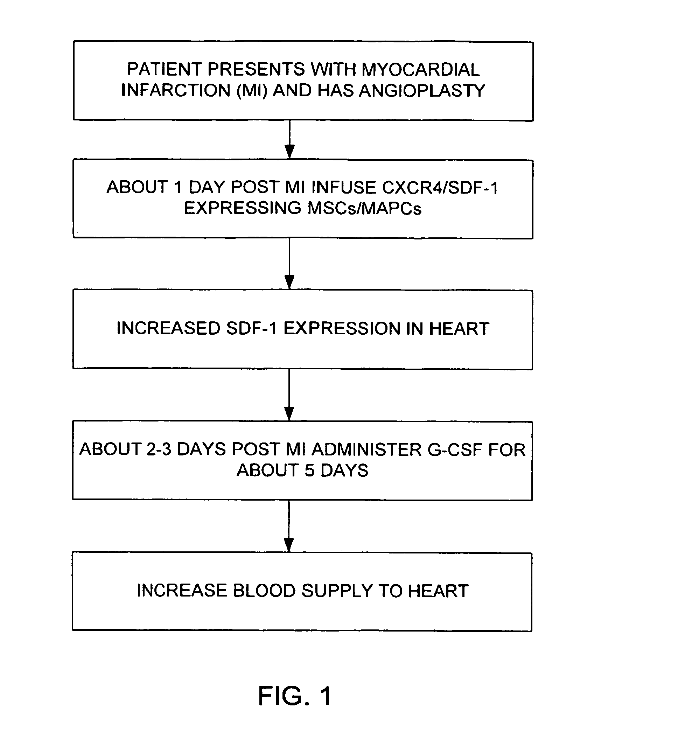

By way of example, FIG. 1 illustrates one clinical strategy using MSCs, MAPCs, and/or other stem cells used to regenerate myocardial structures genetically modified to over-express CXCR4 and SDF-1 to treat a patient with an acute myocardial infarction. In the treatment strategy, a patient with an acute myocardial infarction can be initially treated by anglioplasty. About one day after the myocardial infarction MSCs, MAPCs, and/or other stem cells used to regenerate myocardial structures genetically modified to over-express CXCR4 and SDF-1 are infused into the patient by arterial or venous infusion. The genetically modified MSCs, MAPCs, and/or other stem cells used to regenerate myocardial structures can be autologous and/or allogenic to patient treated and can be harvested, cultured, and genetically modified as previously described. The number of MSCs, MAPCs, and/or other stem cells used to regenerate myocardial structures infused can include, for example, about two million stem cells. This number can be greater or lower depending on the specific application.

The infusion of the genetically modified MSCs, MAPCs, and/or other stem cells used to regenerate myocardial structures increases the SDF-1 expression in the infarcted myocardium. The increased SDF-1 expression promotes MSCs and/or MAPCs survival in the infarcted myocardium as well as homing of other progenitor and stem cells in the peripheral blood to the infarcted myocardium.

About two to about three days following myocardial infarction, GCS-F is administered to the patient for about 5 days. The GCS-F mobilizes additional multipotent progenitor or stem cells from tissue, such as bone marrow, into the blood supply of the patient. The SDF-1 and/or CXCR4 expressed by the MSCs, MAPCs, and/or other stem cells used to regenerate myocardial structures induces these progenitor or stem cells in the peripheral blood to the infarcted myocardium. The MSCs, MAPCs, and/or other stem cells used to regenerate myocardial structures as well as the mobilized progenitor or stem cells induced to the infarcted myocardium can facilitate myocardial regeneration and provide a substantial increase in left ventricle function.

It will be appreciated, that although the genetically modified MSCs, MAPCs, and/or other stem cells are primarily described for treatment of an acute myocardial infarction or congestive heart failure, the genetically modified MSCs, MAPCs, and/or other stem cells in accordance with the present invention can be used for other therapeutic applications. For example, the genetically modified MSC, MAPCs, and/or other stem cells in accordance with the present invention can be used for the regeneration of hepatocytes to replace damaged liver tissue and restore liver function, in conjunction with marrow transplantation, for the regeneration of marrow following marrow ablation by chemotherapy and/or irradiation, for bone and/or cartilage reconstruction, for the correcting of muscle disorders (e.g., muscular dystrophy), as well as other therapeutic applications where tissue is regenerated or where MSCs, MAPCs, and/or other stems are typically used.

### Examples

The present invention is further illustrated by the following series of examples. The examples are provided for illustration and are not to be construed as limiting the scope or content of the invention in any way.

### Effect of CXCR4 expression on MSC homing and survivability

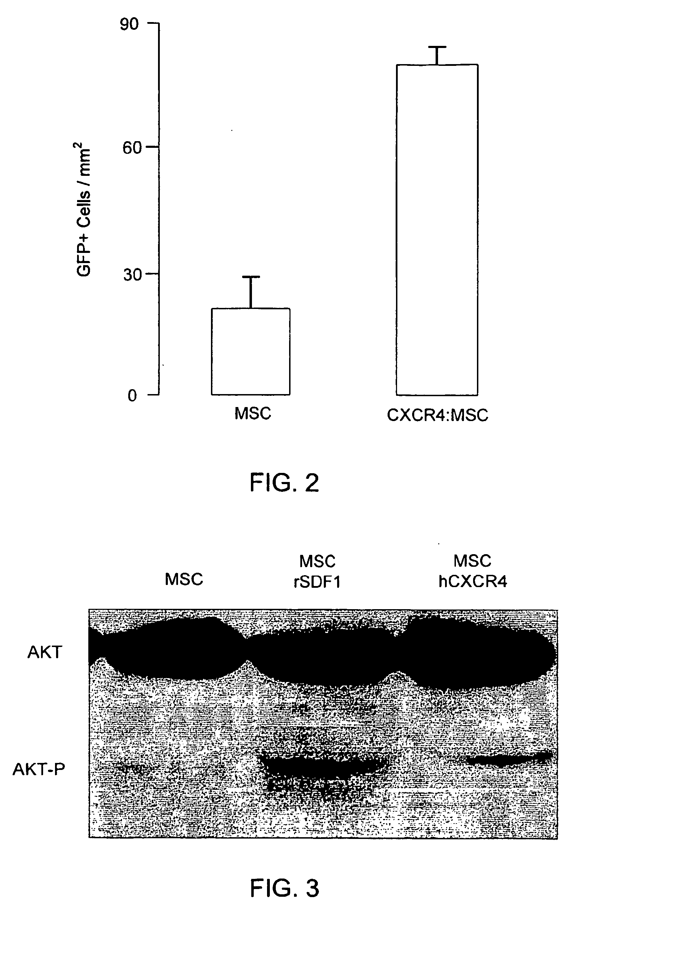

In order to ascertain whether genetically modifying MSCs to express CXCR4 affected the homing and survivability of MSC to infarcted myocardium, 2 million MSCs and MSCs genetically modified to express CXCR4 were infused intravenously in a rat 1 day after myocardial infarction facilitated by left ascending artery ligation. FIG. 2 shows the number of MSCs and MSCs genetically modified to express CXCR4 in the infarct zone per unit area three days after infusion of the MSCs and genetically modified MSCs.

The number of genetically modified MSCs per unit area was substantially greater than the number of MSCs that were not genetically modified. This indicates that MSCs expressing CXCR4 have improved survivability and homing compared to MSC that are not genetically modified.

### CXCR4:SDF-1 axis is Anti-Apoptotic

Western blot analysis for AKT and phosphorylated AKT was performed on MSCs, and MSCs transfected with CXCR4 or SDF-1 to determine if genetic modification of MSCs to express of CXCR4 or SDF-1 has anti-apoptotic effects on the MSCs (i.e., mitigates or inhibits apoptosis ofMSCs) in infarcted myocardium. FIG. 3 shows that the AKT of rat MSCs stably transfected to express CXCR4 or SDF-1 was readily phosphorylated in comparison to the AKT of MSCs not transfected. The phosphorylation of AKT has been found to inhibit apoptosis of MSCs.

### Effect of SDF-1 or CXCR4 expression on MSC homing and survivability

To evaluate whether genetically modifying MSCs to express CXCR4 affected the homing and survivability of MSCs to infarcted myocardium, MSCs and MSCs genetically modified to express CXCR4 or SDF-1 were infused intravenously, respectively, into separate rats after myocardial infarction by LAD ligation.

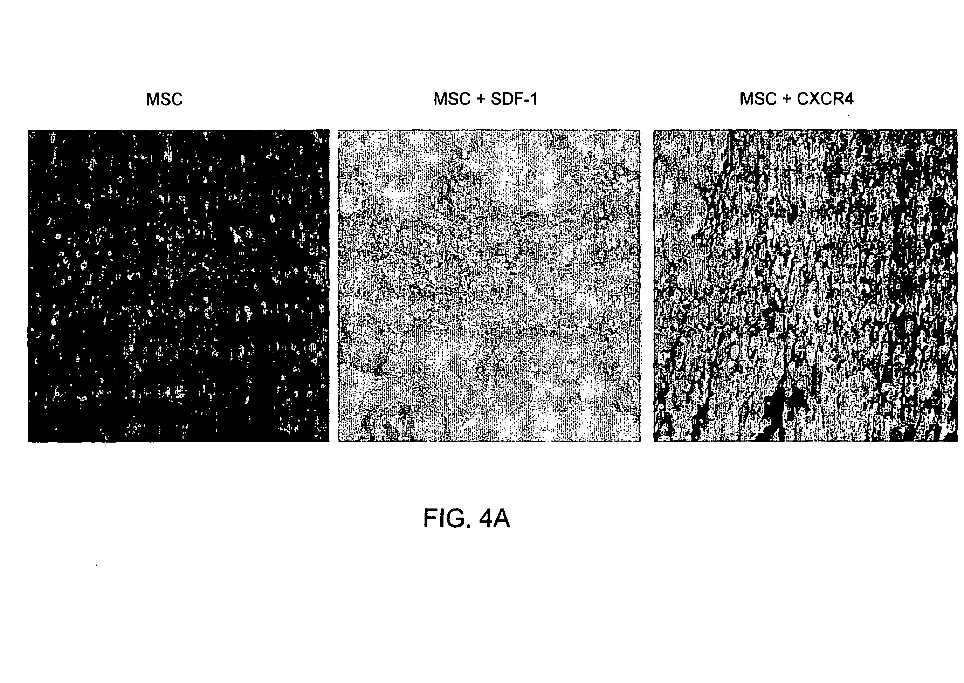

FIG. 4A are photographs showing representative sections of the infarct zone of the respective rats three days following infusion of the control MSCs and the MSCs expressing SDF-1 and CXCR4.

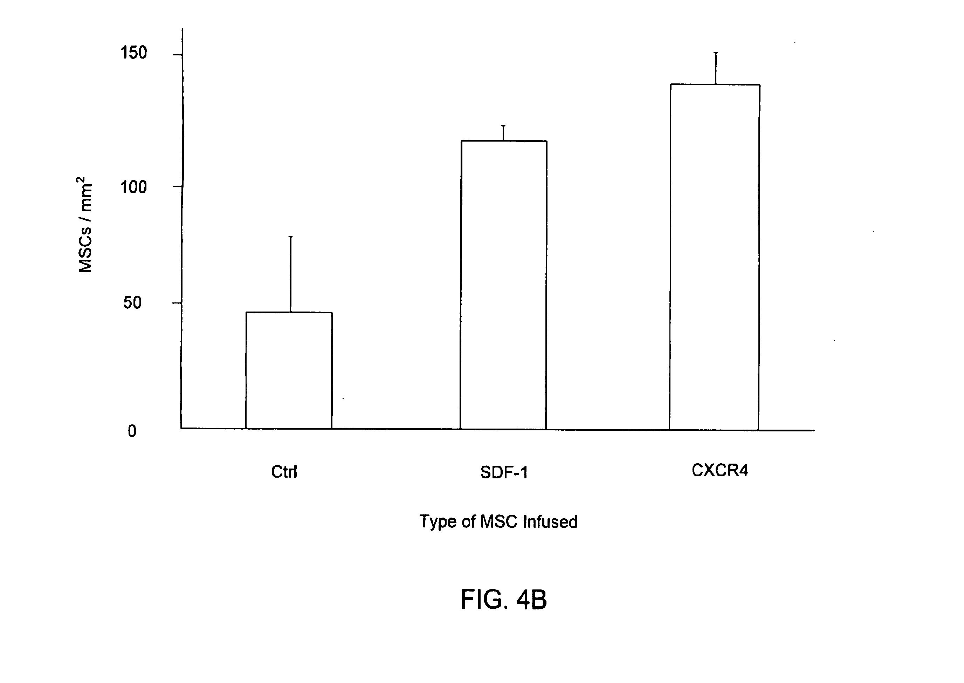

FIG. 4B compares the number of control MSCs with the number of MSCs genetically modified to express CXCR4 or SDF-1 in the infarct zones per unit area three days after infusion.

As can be seen from both FIG. 4A and 4B, the number of genetically modified MSCs per unit area was substantially greater the number of control MSCs. This indicates that MSCs expressing CXCR4 or SDF-1 have improved survivability and homing compared to MSC that are not genetically modified.

### SDF-1 expression will also decrease

### apoptosis in the surviving myocardial tissue

The infarct zone of respective rats treated with control MSCs and MSCs expressing SDF-1 were examined.

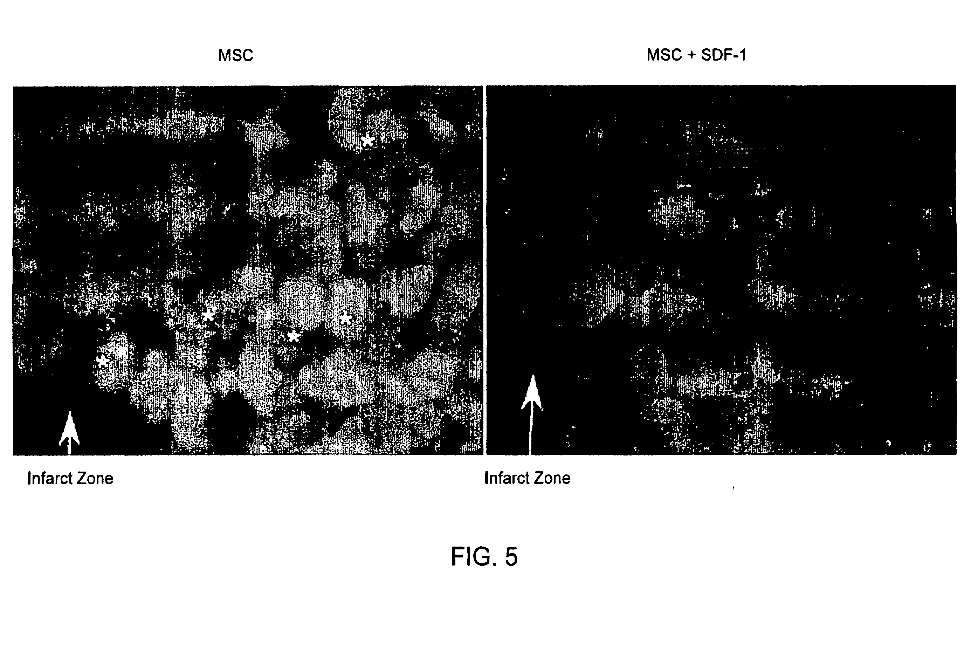

FIG. 5 are photographs of the respective infarct zones four days following myocardial infarction. The photographs indicate that MSCs expressing SDF-1 decrease apoptosis in the surviving myocardial tissue.

### Effect of MSC expressing CXCR4 or SDF-1 on ischemic cardiomyopathy

To evaluate whether genetically modifying MSCs to express CXCR affected the homing and survivability of MSC to infarcted myocardium, MSCs and MSCs genetically modified to express CXCR4 or SDF-1 were infused intravenously, respectively, into separate rats 1 day after myocardial infarction LAD ligation.

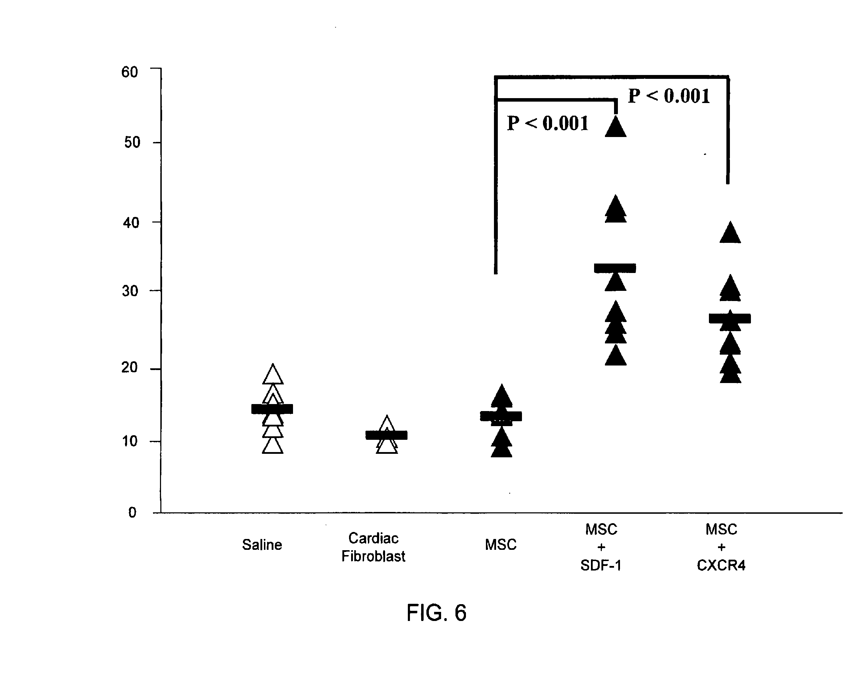

FIG. 6 compares improvement in LV function fourteen days following myocardial infarction for rats implanted with saline, cardiac fibroblast, control MSCs, MSCs expressing SDF-1, and MSCs expressing CXCR4. The infarcted myocardium treated with MSCs expressing SDF-1 or CXCR4 showed substantially increased LV function as measured by shortening fraction. No significant difference was observed in shortening fraction between treatment strategies of transplantation of control MSCs, cardiac fibroblasts, and saline.

## Claims

1. Use of mesenchymal stems cells genetically modified to express CXCR4 in the manufacture of a medicament for treating a myocardial infarction wherein the medicament is intended to be delivered to the infarcted myocardial tissue and the mesenchymal stem cells are not human embryonic stem cells.

2. Mesenchymal stem cells genetically modified to express CXCR4 for treating myocardial infarction, wherein the mesenchymal stem cells are not human embryonic stem cells.

3. Use according to claim 1 or mesenchymal stem cells of claim 2, wherein the stem cells express a protein or a polypeptide with an amino acid sequence comprising at least one of SEQ ID NO: 1 or SEQ ID NO: 2.

4. Use according to claim 1 or mesenchymal stem cells of claim 2, wherein the medicament is intended to be administered by a method selected from the group consisting of direct injection, venous infusion and arterial infusion.

5. Use according to claim 1 or mesenchymal stem cells of claim 2, further comprising the use of a mobilizing agent which induces the mobilization of other stem cells from tissue to peripheral blood in the manufacture of a medicament for treating myocardial infarction.

6. Use according to claim 1 or mesenchymal stem cells of claim 2, wherein the cells are further genetically modified to express SDF-1.

7. Use according to claim 1 or mesenchymal stem cells of claim 2, wherein the stem cells further express a protein or a polypeptide with an amino acid sequence comprising at least one of SEQ ID NO: 5, SEQ ID NO: 6 or SEQ ID NO: 7.

## Patentansprüche

1. Verwendung von mesenchymalen Stammzellen, genetisch modifiziert, um CXCR4 zu exprimieren, in der Herstellung eines Medikaments zum Behandeln eines Myokardinfarkts, wobei das Medikament dem infarzierten Myokardgewebe zugeführt werden soll und die mesenchymalen Stammzellen nicht humane embryonale Stammzellen sind.

2. Mesenchymale Stammzellen, genetisch modifiziert, um CXCR4 zu exprimieren, zum Behandeln von Myokardinfarkt, wobei die mesenchymalen Stammzellen nicht humane embryonale Stammzellen sind.

3. Verwendung gemäß Anspruch 1 oder mesenchymale Stammzellen nach Anspruch 2, wobei die Stammzellen ein Protein oder ein Polypeptid mit einer Aminosäuresequenz, umfassend mindestens eine von SEQ ID NO: 1 oder SEQ ID NO: 2, exprimieren.

4. Verwendung gemäß Anspruch 1 oder mesenchymale Stammzellen nach Anspruch 2, wobei das Medikament durch ein Verfahren, ausgewählt aus der Gruppe, bestehend aus direkter Injektion, venöser Infusion und arterieller Infusion, verabreicht werden soll.

5. Verwendung gemäß Anspruch 1 oder mesenchymale Stammzellen nach Anspruch 2, weiterhin umfassend die Verwendung eines Mobilisierungsmittels, welches die Mobilisierung anderer Stammzellen aus Gewebe zu peripherem Blut induziert, in der Herstellung eines Medikaments zum Behandeln von Myokardinfarkt.

6. Verwendung gemäß Anspruch 1 oder mesenchymale Stammzellen nach Anspruch 2, wobei die Zellen weiterhin genetisch modifiziert sind, um SDF-1 zu exprimieren.

7. Verwendung gemäß Anspruch 1 oder mesenchymale Stammzellen nach Anspruch 2, wobei die Stammzellen weiterhin ein Protein oder ein Polypeptid mit einer Aminosäuresequenz, umfassend mindestens eine von SEQ ID NO: 5, SEQ ID NO: 6 oder SEQ ID NO: 7, exprimieren.

## Revendications

1. Utilisation de cellules souches mésenchymateuses génétiquement modifiées pour exprimer CXCR4 dans la fabrication d'un médicament pour le traitement de l'infarctus du myocarde, où le médicament est destiné à être administré au tissu myocardique touché par l'infarctus et les cellules souches mésenchymateuses ne sont pas des cellules souches embryonnaires humaines.

2. Cellules souches mésenchymateuses génétiquement modifiées pour exprimer CXCR4 pour le traitement de l'infarctus du myocarde, où les cellules souches mésenchymateuses ne sont pas des cellules souches embryonnaires humaines.

3. Utilisation selon la revendication 1 ou cellules souches mésenchymateuses selon la revendication 2, où les cellules souches expriment une protéine ou un polypeptide ayant une séquence d'acides aminés comprenant au moins une séquence parmi SEQ ID NO:1 1 ou SEQ ID NO:2.

4. Utilisation selon la revendication 1 ou cellules souches mésenchymateuses selon la revendication 2, où le médicament est destiné à être administré par une méthode choisie dans le groupe constitué d'une injection directe, d'une perfusion veineuse et d'une perfusion artérielle.

5. Utilisation selon la revendication 1 ou cellules souches mésenchymateuses selon la revendication 2, comprenant en outre l'utilisation d'un agent mobilisateur qui induit la mobilisation d'autres cellules souches depuis le tissu vers le sang périphérique dans la fabrication d'un médicament pour le traitement de l'infarctus du myocarde.

6. Utilisation selon la revendication 1 ou cellules souches mésenchymateuses selon la revendication 2, où les cellules sont en outre génétiquement modifiées pour exprimer SDF-1.

7. Utilisation selon la revendication 1 ou cellules souches mésenchymateuses selon la revendication 2, où les cellules souches expriment en outre une protéine ou un polypeptide ayant une séquence d'acides aminés comprenant au moins une séquence parmi SEQ ID NO:5, SEQ ID NO:6 ou SEQ ID NO:7.
